# EUROPEAN PATENT APPLICATION

(11) **EP 4 643 858 A1**
(43) Date of publication of application: **05.11.2025**
(21) Application number: 24305667.8
(22) Date of filing: 29.04.2024
(51) Int. Cl.: A61K 31/4439, A61K 31/5377, A61P 15/00, A61P 35/00

(54) **METHODS AND PHARMACEUTICAL COMPOSITION FOR THE TREATMENT OF UTERINE DISEASE**

(71) Applicant: Institut National de la Santé et de la Recherche Médicale, 75013 Paris (FR); Centre National de la Recherche Scientifique, 75016 Paris (FR); APHP (Assistance Publique - Hôpitaux de Paris), 75012 Paris (FR); Université Paris Cité, 75006 Paris (FR)
(72) Inventor: The designation of the inventor has not yet been filed
(74) Representative: Cabinet Grosset-Fournier & Demachy

(57) **Abstract**

Control tissues and different lesions of either adenomyosis or endometriosis were exposed in vitro during 24 hours either to vehicle (DMSO) or different doses of BYL719 or ST420, a PIK3CA inhibitor. Inventors observed that lesions treated with DMSO had a higher recruitment of the PIK3CA pathway compared to healthy endometrium.

Importantly, both inhibitors were able to shut down the phosphorylation of AKT. They concluded that PIK3CA inhibition is a good therapeutic target for patients with endometriosis and adenomyosis.

They also investigated the role of the PIK3CA pathway in myoma, another benign uterine condition. They observed that either alpelisib or ST420 were able to inhibit the pathway in myoma. They concluded that myoma demonstrate PIK3CA/AKT pathway activation with somatic mutation and are accessible to targeted treatment.

Accordingly, the invention relates to method for treating uterine disease in a subject in need thereof comprising a step of administrating the subject with a therapeutically effective amount of PIK3CA inhibitor such as compound of formula (I), or a deuterated or tritiated form of the compound of formula (I), BYL719.

## Description

### FIELD OF THE INVENTION:

The present invention relates to use of inhibitors PIK3CA in the treatment of uterine disease, more particularly endometriosis and myoma.

### BACKGROUND OF THE INVENTION:

Endometriosis affects 180 million women worldwide. In France, despite the lack of quantitative data, it is estimated that between 1.5 and 2.5 million women of reproductive age suffer from this condition¹. Globally, including direct costs (with existing symptomatic treatments) and indirect costs, the estimated global societal cost of endometriosis is around $70 billion per year¹. Therefore, this condition has significant social, economic, and public health repercussions.

There is a major medical need, accentuated by the significant impact of this condition on the quality of life of patients. Acute pain and infertility (30-40% of women with endometriosis experience infertility, and 50% of women with infertility may have endometriosis) are the main symptoms of the disease¹. In addition to acute pain, fatigue, depression, anxiety, and infertility are very common, and in some women, endometriosis causes debilitating pain that prevents them from working or studying¹.

Endometriosis is a common hormonally dependent gynecological condition that develops in women of reproductive age. It is secondary to an abnormal migration of endometrial tissue outside the uterine cavity. This ectopic implantation (on the ovaries, peritoneum, or along the digestive tract) causes local inflammation characterized clinically by varying degrees of pain, fertility disorders, with a major impact on quality of life, and limited therapeutic options. Of note, when the endometrial tissue does not migrate outside the uterine cavity but instead grows deep in the muscular wall of the uterus it is called adenomyosis¹.

The pathophysiology of endometriosis or adenomyosis is poorly understood, combining genetic and environmental factors, but also possibly somatic tissue mutations within the endometrium². Few publications highlight the activation of many cellular pathways including the PIK3CA/AKT/mTOR, that is involved in cell growth, proliferation, metabolism, and migration².

Current treatments are mainly symptomatic - they include pain relief medications, hormonal contraceptives, surgeries to remove endometriosis lesions (laparoscopy), and in some cases, hysterectomy (100,000 hysterectomies per year are performed in the US for endometriosis)². The main candidate drugs in development or recently approved are hormonal treatments: hormonal contraceptives, and GnRH agonists or antagonists (inducing 'artificial menopause')². None of these approaches target the molecular and cellular mechanisms involved in the pathophysiology of the disease. Therefore, they transiently relieve symptoms during treatment, without reducing lesions or reducing the occurrence of lesion recurrence after treatment cessation. Adverse effects are numerous, including menopause syndromes (including bone demineralization).

Phosphatidylinositol 3-kinases (PI3Ks) comprise a family of lipid kinases that phosphorylate phosphatidylinositides at the 3' position of the inositol ring. They have been divided into three classes based on their substrate specificity and sequence homology.

Class I PI3Ks phosphorylate phosphatidylinositol-4,5-diphosphate (PIP₂) downstream of either receptor tyrosine kinases or G-protein coupled receptors to form the second messenger phosphatidylinositol-3,4,5-trisphosphate (PIP₃), which signals for increased cell growth, metabolism, and cell-cycle progression. Class I consists of four family members, each of which forms a heterodimer between a catalytic subunit, p110, and a regulatory subunit. The family is further subdivided into Class IA, where isoforms p110α, ρ110β and p110δ form heterodimers with the p85 family of regulatory subunits, and Class IB, where p110γ is the sole member and forms a heterodimer with either the p87 or p101 regulatory subunit.

Each isoform has been shown to have overlapping, but non-redundant physiological roles. PI3K-α and β are both ubiquitously expressed. PI3K-α plays a key role in glucose homeostasis and insulin signaling and is involved in driving myocardial growth via PIP₃ dependent pathways. PI3K-β, in contrast, has been shown to regulate the activity of the platelet integrin α_{IIb}β₃ in the context of platelet adhesion and aggregation. As such PI3K-β is under investigation as a novel treatment for thrombosis, and the first in-human trials have shown promising results. PI3K-γ and δ have more restricted expression, largely limited to the hematopoietic system. They both play important, non-redundant roles in the immune system, and so are both under consideration as immune modulatory targets. PI3K-γ inhibition is being pursued for rheumatoid arthritis and asthma, and PI3K-δ for activated PI3K-δ syndrome (APDS).

Their diverse functions notwithstanding, PI3Ks are perhaps most well-known as oncology targets. The PI3K pathway is one of the most frequently dysregulated in cancer.

Thus, several inhibitors of PI3Ks have been developed that inhibit multiple class 1A PI3K isoforms and commonly known as "pan-PI3K" inhibitors.

Currently, alpelisib is the only approved PI3KCA inhibitor for breast cancer³ and PIK3CA-related overgrowth syndromes^{4,5} (the latter indication was obtained through our work). However, alpelisib has a major drawback of being associated with insulin resistance which can lead to diabetes in about 30% of patients. This side effect is not tolerable in the case of endometriosis treatment.

### SUMMARY OF THE INVENTION:

Control tissues (healthy endometrium) and different lesions of either adenomyosis or endometriosis were exposed in vitro during 24 hours either to vehicle (DMSO) or different doses of BYL719 or ST420 a PIK3CA inhibitor. Inventors observed that lesions treated with DMSO had a higher recruitment of the PIK3CA pathway compared to healthy endometrium. Importantly, both inhibitors were able to shut down the phosphorylation of AKT. They concluded that PIK3CA inhibition is a good therapeutic target for patients with endometriosis and adenomyosis.

They also investigated the role of the PIK3CA pathway in myoma (or uterine fibroids), another benign uterine condition. They observed that either alpelisib or ST420 were able to inhibit the pathway in myoma. They concluded that myoma demonstrate PIK3CA/AKT pathway activation with somatic mutation and are accessible to targeted treatment.

### DETAILED DESCRIPTION OF THE INVENTION:

Accordingly, **in a first aspect,** the present invention relates to a method for treating uterine disease in a subject in need thereof comprising a step of administrating the subject with a therapeutically effective amount of PI3K inhibitor, in particular PIK3CA inhibitor.

In some embodiments, the present invention also relates to a method for treating uterine disease in a subject in need thereof, wherein the method consists essentially in a step of administrating the subject with a therapeutically effective amount of PIK3CA inhibitor.

In some embodiments, the present invention also relates to a method for treating uterine disease in a subject in need thereof, comprising a step of administrating the subject with a therapeutically effective amount of PIK3CA inhibitor.

As used herein, the terms **"treating"** or **"treatment"** refer to both prophylactic or preventive treatment as well as curative or disease modifying treatment, including treatment of subject at risk of contracting the disease or suspected to have contracted the disease as well as subject who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein, the term **"uterine disease"** refers to are conditions/disorders that affect the uterus such as endometriosis, which is when tissue grows where it's not supposed to.

In a particular embodiment, the uterine disease is selected from the group consisting of but not limited to: endometriosis, myoma, adenomyosis, uterine fibroids or endometrial cancer.

In a particular embodiment, the uterine disease is endometriosis.

As used herein, the term **"endometriosis"** refers to a common hormonally dependent gynecological condition that develops in women of reproductive age. It is secondary to an abnormal migration of endometrial tissue outside the uterine cavity. This ectopic implantation (on the ovaries, peritoneum, or along the digestive tract) causes local inflammation characterized clinically by varying degrees of pain, fertility disorders, with a major impact on quality of life, and limited therapeutic options. When the endometrial tissue does not migrate outside the uterine cavity but instead grows deep in the muscular wall of the uterus it is called adenomyosis.

In a particular embodiment, the uterine disease is myoma.

As used herein, the term **"myoma"** refers to non-cancerous tumors that may develop in or around the uterus. Made partly of muscle tissue, myomas seldom develop in the cervix, but when they do, there are usually myomas in the larger, upper part of the uterus as well. Myomas in this part of the uterus are also called fibroids or leiomyomas.

As used herein, the term **"subject"** refers to any mammals, such as a rodent, a feline, a canine, and a primate. Particularly, in the present invention, the subject is a human afflicted with or susceptible to be afflicted with at least one of uterine disease as described above.

In a particular embodiment, the subject is a human afflicted with or susceptible to be afflicted with endometriosis.

In another embodiment, the subject is a human afflicted with or susceptible to be afflicted with myoma.

As used herein, the term **"PI3K"** refers to phosphoinositide 3-kinases also called phophatidylinositide 3-kinases. PI3K belongs to a family of enzymes which phosphorylate the 3'hydroxyl group of the inositol ring of the phosphatidylinositol (PtdIns). The PI3K signalling pathway can be activated, resulting in the synthesis of PIP3 from PIP2. PIK3CA is mainly recruited through tyrosine kinase receptors. **PIK3CA** encodes the 110-kDa catalytic alpha subunit of PI3K (p110α), which converts, at the plasma membrane, phosphatidylinositol 4,5-bisphosphate (PtdIns(4,5)P2) to phosphatidylinositol 3,4,5-trisphosphate (PtdIns(3,4,5)P3; or PIP3) with subsequent recruitment of PDK1, which in turn phosphorylates AKT on the Thr308 residue to initiate downstream cellular effects. PIK3CA also regulates many other pathways, including the Rho/Rac1 signaling cascade.

As used herein, the term **"PIK3CA inhibitor"** refers to a natural or synthetic compound that has a biological effect to inhibit the activity or the expression of PI3K. More particularly, such compound is capable of inhibiting the kinase activity of at least one member of PI3K family, for example, at least a member of Class I PI3K.

In a particular embodiment, said PI3K inhibitor may be a pan-inhibitor of Class I PI3K (known as p110) or isoform specific of Class I PI3K isoforms (among the four types of isoforms, p110α, p110β, p110γ or p110δ).

In a particular embodiment, the PIK3CA inhibitor is a peptide, peptidomimetic, small organic molecule, antibody, aptamers, siRNA or antisense oligonucleotide.

The term "peptidomimetic" refers to a small protein-like chain designed to mimic a peptide.

In a particular embodiment, the inhibitor of PI3K is an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity.

In a particular embodiment, the PIK3CA inhibitor is a small organic molecule.

The term **"small organic molecule"** refers to a molecule of a size comparable to those organic molecules generally used in pharmaceuticals. The term excludes biological macromolecules (e.g., proteins, nucleic acids, etc.). Preferred small organic molecules range in size up to about 5000 Da, more preferably up to 2000 Da, and most preferably up to about 1000 Da.

In a particular embodiment, the PI3K inhibitor is a small molecule which is an isoform-selective inhibitor of PI3K selected among the following compounds: BYL719 (Alpelisib, Novartis), GDC-0032 (Taselisib, Genentech/Roche), BKM120 (Buparlisib), TAK-117/MLN1117/INK1117 (Serabelisib), A66 (University of Auckland - CAS No. : 1166227-08-2), GSK260301 (Glaxosmithkline), KIN-193 (Astra-Zeneca - CAS No. : 1173900-33-8), TGX221 (Monash University - CAS No. : 663619-89-4), TG-1202 (Umbralisib), CAL101 (Idelalisib, Gilead Sciences), GS-9820 (Acalisib, Gilead Sciences), AMG319 (Amgen - CAS No. 1608125-21-8), IC87114 (Icos Corporation - CAS No. : 371242-69-2), BAY80-6946 (Copanlisib, Bayer Healthcare), GDC0941 (Pictilisib, Genentech), IPI145 (Duvelisib, Infinity), SAR405 (Sanofi - CAS No. 1523406-39-4), PX-866 (Sonolisib, Oncothyreon), perifosine, BEZ235 (Dactolisib), CUDC-907 (Fimepinostat), SAR245409/XI,765 (Voxtalisib), XL-147 (Pilaralisib), GDC-0077 (Inavolisib), AZD-8186 (Astra-Zeneca - CAS No. 1627494-13-6), IPI549 (Eganelisib) or their pharmaceutically acceptable salts. In a more particular embodiment, the isoform-selective inhibitor of PI3K is selected among the following compounds: BYL719 (Alpelisib, Novartis), A66 (University of Auckland), GDC-0077 (Inavolisib, Genentech/Roche), CYH33 (Risovalisib), TAK-117/MLN1117/INK1117 (Serabelisib) or their pharmaceutically acceptable salts.

In an even more particular embodiment, the isoform-selective inhibitor of PI3K is selected among the following compounds: a compound of formula (I), or a deuterated or tritiated form of the compound of formula (I), BYL719 (Alpelisib, Novartis), GDC-0077 (Inavolisib, Genentech/Roche), TAK-117/MLN1117/INK1117 (Serabelisib) or their pharmaceutically acceptable salts.

Such PI3K inhibitors are well-known in the art and described for example in Wang et al Acta Pharmacological Sinica (2015) 36: 1170-1176.

In a particular embodiment, the PI3K inhibitor is BYL719 and its derivatives.

As used herein, the term "BYL 719" also called alpelisib is an ATP-competitive oral PI3K inhibitor selective for the p110α isoform that is activated by a mutant PIK3CA gene (Furet P., et al. 2013; Fritsch C., et al 2014). This molecule is also called Alpelisib and has the following formula and structure in the art C₁₉H₂₂F₃N₅O₂S:

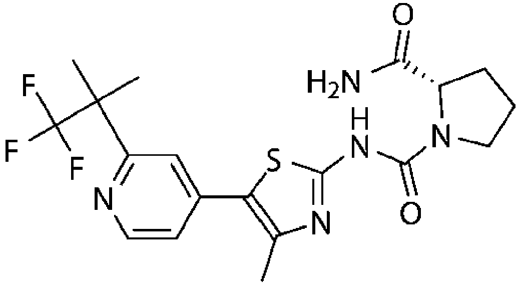

In a particular embodiment, the PI3K inhibitor is GDC-0032 and its derivatives, developed by Roche. This molecule also called Taselisib has the following formula and structure in the art C₂₄H₂₈N₈O₂:

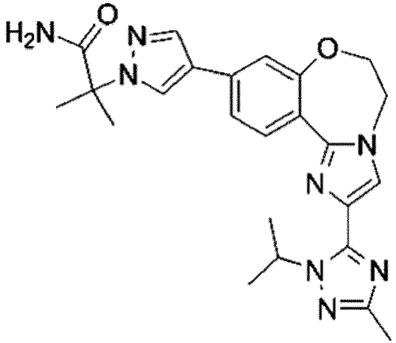

In a particular embodiment, the PIK3CA inhibitor is compounds of formula (I), or a deuterated or tritiated form of the compound of formula (I), or pharmaceutically acceptable salts thereof: 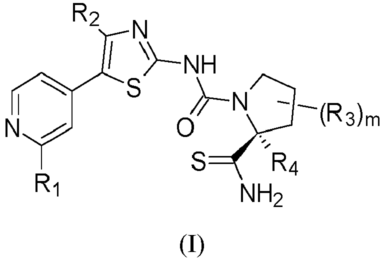 wherein:
- R₁ is a (C₁-C₆)alkyl group or a (C₃-C₆)cycloalkyl group, unsubstituted or substituted with one or more fluorine atoms;
- R₂ is chosen from:
   ▪ a hydrogen atom, and
   ▪ a (C₁-C₆)alkyl group,
- m is 0, 1 or 2;
- each R₃, when present, is independently chosen from:
   ▪ a fluorine atom,
   ▪ a (C₁-C₆)alkyl group, unsubstituted or substituted with one or more halogen atoms,
   ▪ a (C₃-C₆)cycloalkyl group, unsubstituted or substituted with one or more halogen atoms,
   ▪ a hydroxy group,
   ▪ a (C₁-C₆)alkoxy group, and
   ▪ a -NRR' group, R and R' being independently chosen from a hydrogen atom and a (C₁-C₆)alkyl group,
   or two R₃, that are borne by the same carbon atom, form with the carbon atom bearing them a (C₃-C₆)cycloalkyl ring, unsubstituted or substituted with one or more fluorine atoms; and
- R₄ is chosen from:
   ▪ a fluorine atom,
   ▪ a hydrogen atom,
   ▪ a (C₁-C₆)alkyl group, unsubstituted or substituted with one or more halogen atoms, and
   ▪ a (C₃-C₆)cycloalkyl group, unsubstituted or substituted with one or more halogen atoms,
or R₃ and R₄, when they are borne by two adjacent carbon atoms, form with the carbon atoms bearing them a (C₃-C₆)cycloalkyl ring, unsubstituted or substituted with one or more halogen atom.

Among the compounds of formula (I), mention may be made of the compounds for which R₁ is a (C₁-C₆)alkyl group, in particular a (C₁-C₄)alkyl group, and more particularly a *tert*-butyl group, unsubstituted or substituted with one or more fluorine atoms.
In an embodiment, in the compounds of formula (I), the R₁ group is a *tert*-butyl group substituted with one to three fluorine atoms.
In another embodiment, in the compounds of formula (I), the R₁ group is an unsubstituted *tert*-butyl group.

Among the compounds of formula (I), mention may be made of the compounds for which R₂ is a (C₁-C₆)alkyl group, in particular a (C₁-C₄)alkyl group and more particularly a methyl group.

In an embodiment, mention may be made of the compounds of formula (I) for which m is 0.

In another embodiment, mention may be made of the compounds of formula (I) for which m is 1 or 2, in particular for which m is 1.

In an embodiment, in the compounds of formula (I), m is 1 or 2, and each R₃ is independently chosen from:
▪ a fluorine atom,
▪ a (C₁-C₆)alkyl group, in particular a (C₁-C₄)alkyl group and more particularly a methyl group, unsubstituted or substituted with one or more halogen atoms,
▪ a (C₃-C₆)cycloalkyl group unsubstituted or substituted with one or more halogen atoms, especially one or more fluorine atom;
▪ a hydroxy group,
▪ a (C₁-C₆) alkoxy group, in particular a (C₁-C₄) alkoxy group and more particularly a methoxy group, and
▪ a -NH₂ group.

In an embodiment, in the compounds of formula (I), m is 1 or 2, and each R₃ is independently chosen from:
▪ a fluorine atom,
▪ a (C₁-C₆)alkyl group, in particular a (C₁-C₄)alkyl group and more particularly a methyl group, unsubstituted or substituted with one or more halogen atoms; for example a methyl group or a trifluoromethyl group;
▪ a (C₃-C₆)cycloalkyl group unsubstituted or substituted with one or more halogen atoms, especially one or more fluorine atom;
▪ a hydroxy group, and
▪ a (C₁-C₆) alkoxy group, in particular a (C₁-C₄) alkoxy group and more particularly a methoxy group.

In a particular embodiment, m is 1 or 2, and each R₃ is independently chosen from:
▪ a fluorine atom,
▪ a (C₁-C₆)alkyl group, in particular a (C₁-C₄)alkyl group and more particularly a methyl group, unsubstituted or substituted with one or more halogen atoms; for example a methyl group or a trifluoromethyl group;
▪ a hydroxy group, and
▪ a (C₁-C₆) alkoxy group, in particular a (C₁-C₄) alkoxy group and more particularly a methoxy group.

In another embodiment, in the compounds of formula (I), m is 2 and two R₃, that are borne by the same carbon atom, form with the carbon atom bearing them a (C₃-C₆)cycloalkyl ring, in particular a cyclopropyl ring, unsubstituted or substituted with one or more fluorine atoms.

Among the compounds of formula (I), mention may be made of the compounds for which R₄ is chosen from:
▪ a hydrogen atom, and
▪ a (C₁-C₆)alkyl group, in particular a (C₁-C₄)alkyl group and more particularly a methyl group, unsubstituted or substituted with one or more halogen atoms, in particular with one or more fluorine atoms.

All these sub-groups and specific embodiments, taken alone or in combination, are part of the description.

In an embodiment, mention may be made of the compounds of formula (I) for which:
- R₁ is a (C₁-C₆)alkyl group or a (C₃-C₆)cycloalkyl group, unsubstituted or substituted with one or more fluorine atoms;
- R₂ is chosen from:
   ▪ a hydrogen atom, and
   ▪ a (C₁-C₆)alkyl group;
- m is 0, 1 or 2;
- each R₃, when present, is independently chosen from:
   ▪ a fluorine atom,
   ▪ a (C₁-C₆)alkyl group, unsubstituted or substituted with one or more halogen atoms,
   ▪ a (C₃-C₆)cycloalkyl group unsubstituted or substituted with one or more halogen atoms,
   ▪ a hydroxy group, and
   ▪ a (C₁-C₆) alkoxy group, or two R₃, that are borne by the same carbon atom, form with the carbon atom bearing them a (C₃-C₆)cycloalkyl ring, unsubstituted or substituted with one or more fluorine atoms; and
- R₄ is chosen from:
   ▪ a fluorine atom,
   ▪ a hydrogen atom, and
   ▪ a (C₁-C₆)alkyl group, unsubstituted or substituted with one or more halogen atoms.

In another embodiment, mention may be made of the compounds of formula (I) for which:
- R₁ is a (C₁-C₆)alkyl group, in particular a (C₁-C₄)alkyl group, unsubstituted or substituted with one to three fluorine atoms; and more particularly a *tert*-butyl group, unsubstituted or substituted with one to three fluorine atoms;
- R₂ is a (C₁-C₆)alkyl group, in particular a (C₁-C₄)alkyl group and more particularly a methyl group,
- m is 0, 1 or 2;
- each R₃, when present, is independently chosen from:
   ▪ a fluorine atom,
   ▪ a (C₁-C₆)alkyl group, in particular a (C₁-C₄)alkyl group and more particularly a methyl,
   ▪ a hydroxy group, and
   ▪ a (C₁-C₆) alkoxy group, in particular a (C₁-C₄) alkoxy group and more
   particularly a methoxy group,
   or two R₃, that are borne by the same carbon atom, form with the carbon atom bearing them a (C₃-C₆)cycloalkyl ring, in particular a cyclopropyl ring, unsubstituted or substituted with one or two fluorine atoms; and
- R₄ is chosen from:
   ▪ a hydrogen atom, and
   ▪ a (C₁-C₆)alkyl group, in particular a (C₁-C₄)alkyl group and more particularly a methyl group.

In another embodiment, mention may be made of the compounds of formula (I) for which:
- R₁ is a (C₁-C₆)alkyl group, in particular a (C₁-C₄)alkyl group and more particularly a *tert*-butyl group, substituted with one to three fluorine atoms;
- R₂ is a (C₁-C₆)alkyl group, in particular a (C₁-C₄)alkyl group and more particularly a methyl group,
- m is 0 or 1;
- R₃, when present, is chosen from:
   ▪ a hydroxy group, and
   ▪ a (C₁-C₆) alkoxy group, in particular a (C₁-C₄) alkoxy group and more particularly a methoxy group,
   preferably R₃ is a hydroxy group; and
- R₄ is a hydrogen atom.

The nomenclature of the following compounds (1) to (20) was generated according to the principles of the International Union of Pure and Applied Chemistry, using IUPAC rules for organic compounds.

Among the compounds of formula (I), mention may be made in particular of the following compounds, or a deuterated or tritiated form of the compound of formula (I), or a pharmaceutically acceptable salt thereof:
(1) (S)-2-carbamothioyl-4,4-difluoro-*N*-(4-methyl-5-(2-(1,1,1-trifluoro-2-methylpropan-2-yl)pyridin-4-yl)thiazol-2-yl)pyrrolidine-1-carboxamide,
(2) (2S,4R)-2-carbamothioyl-4-fluoro-*N*-(4-methyl-5-(2-(1,1,1-trifluoro-2-methylpropan-2-yl)pyridin-4-yl)thiazol-2-yl)pyrrolidine-1-carboxamide,
(3) (2S,4S)-2-carbamothioyl-4-fluoro-*N-*(4-methyl-5-(2-(1,1,1-trifluoro-2-methylpropan-2-yl)pyridin-4-yl)thiazol-2-yl)pyrrolidine-1-carboxamide,
(4) (S)-6-carbamothioyl-*N*-(4-methyl-5-(2-(1,1,1-trifluoro-2-methylpropan-2-yl)pyridin-4-yl)thiazol-2-yl)-1,1-difluoro-5-azaspiro[2,4]heptane-5-carboxamide,
(5) (S)-2-carbamothioyl-*N*-(4-methyl-5-(2-(1,1,1-trifluoro-2-methylpropan-2-yl)pyridin-4-yl)thiazol-2-yl)pyrrolidine-1-carboxamide,
(6) (S)-2-carbamothioyl-2-methyl-*N*-(4-methyl-5-(2-(1,1,1-trifluoro-2-methylpropan-2-yl)pyridin-4-yl)thiazol-2-yl)pyrrolidine-1-carboxamide,
(7) (2S,4R)-2-carbamothioyl-4-methoxy-*N*-(4-methyl-5-(2-(1,1,1-trifluoro-2-methylpropan-2-yl)pyridin-4-yl)thiazol-2-yl)pyrrolidine-1-carboxamide,
(8) (2S,4S)-2-carbamothioyl-4-methoxy-*N*-(4-methyl-5-(2-(1,1,1-trifluoro-2-methylpropan-2-yl)pyridin-4-yl)thiazol-2-yl)pyrrolidine-1-carboxamide,
(9) (2S,5R)-2-carbamothioyl-5-methyl-*N*-(4-methyl-5-(2-(1,1,1-trifluoro-2-methylpropan-2-yl)pyridin-4-yl)thiazol-2-yl)pyrrolidine-1-carboxamide,
(10) (2S,5S)-2-carbamothioyl-5-methyl-*N*-(4-methyl-5-(2-(1,1,1-trifluoro-2-methylpropan-2-yl)pyridin-4-yl)thiazol-2-yl)pyrrolidine-1-carboxamide,
(11) (2S,4S)-2-carbamothioyl-4-methyl-N-(4-methyl-5-(2-(1,1,1-trifluoro-2-methylpropan-2-yl)pyridin-4-yl)thiazol-2-yl)pyrrolidine-1-carboxamide,
(12) (2S,4S)-2-carbamothioyl-N-(4-methyl-5-(2-(1,1,1-trifluoro-2-methylpropan-2-yl)pyridin-4-yl)thiazol-2-yl)-4-(trifluoromethyl)pyrrolidine-1-carboxamide,
(13) (2S,4R)-2-carbamothioyl-4-hydroxy-N-(4-methyl-5-(2-(1,1,1-trifluoro-2-methylpropan-2-yl)pyridin-4-yl)thiazol-2-yl)pyrrolidine-1-carboxamide,
(14) (2S,3S)-2-carbamothioyl-3-hydroxy-N-(4-methyl-5-(2-(1,1,1-trifluoro-2-methylpropan-2-yl)pyridin-4-yl)thiazol-2-yl)pyrrolidine-1-carboxamide,
(15) (2S,3 S)-2-carbamothioyl-3-methoxy-N-(4-methyl-5-(2-(1,1,1-trifluoro-2-methylpropan-2-yl)pyridin-4-yl)thiazol-2-yl)pyrrolidine-1-carboxamide,
(16) (S)-6-carbamothioyl-N-(4-methyl-5-(2-(1,1,1-trifluoro-2-methylpropan-2-yl)pyridin-4-yl)thiazol-2-yl)-5-azaspiro[2.4]heptane-5-carboxamide,
(17) (2S,4S)-2-carbamothioyl-4-hydroxy-N-(4-methyl-5-(2-(1,1,1-trifluoro-2-methylpropan-2-yl)pyridin-4-yl)thiazol-2-yl)pyrrolidine-1-carboxamide;
(18) (S)-2-carbamothioyl-*N*-(4-methyl-5-(2-tert-butyl-pyridin-4-yl)thiazol-2-yl)pyrrolidine-1-carboxamide,
(19) (2S,4S)-2-carbamothioyl-4-fluoro-*N*-(4-methyl-5-(2-tert-butyl-pyridin-4-yl)thiazol-2-yl)pyrrolidine-1-carboxamide, and
(20) (S)-6-carbamothioyl-*N*-(4-methyl-5-(2-tert-butyl-pyridin-4-yl)thiazol-2-yl)-5-azaspiro[2.4]heptane-5-carboxamide.

Among the preceding listed compounds, the following compounds may particularly be cited: (5), (7), (8), (13), (14) and (17), or a deuterated or tritiated form of the compound of formula (I), or a pharmaceutical salt thereof.

Among the preceding listed compounds, the following compounds may particularly be cited: (5), (13), (14) and (17), in particular (5), (13) and (14), or a deuterated or tritiated form of the compound of formula (I), or a pharmaceutical salt thereof.

In another embodiment, the inhibitor of PIK3CA is the intermediate compounds of formulae (I-A) and (II-A) or, or a deuterated or tritiated form of the compound of formula (I), any of their pharmaceutically acceptable salts: 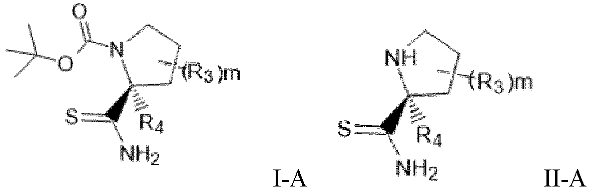 wherein R₃, R₄ and m are as defined above; or R₃ represents a protected hydroxy group, for example a hydroxy group protected with a *tert*-butyldiphenylsilyl ether (tBDPS) protecting group.

In particular, in intermediate compounds of formula (I-A) or (II-A), m, R₃ and R₄ may have any one of the specific definitions as mentioned above for the compounds of formula (I).

In an embodiment, in the intermediate compounds of formula (I-A) or (II-A) :
- m is 0, 1 or 2,
- each R₃, when present, is independently chosen from:
   ▪ a fluorine atom,
   ▪ a (C₁-C₆)alkyl group, in particular a (C₁-C₄)alkyl group and more particularly a methyl group, unsubstituted or substituted with one or more halogen atoms,
   ▪ a (C₃-C₆)cycloalkyl group unsubstituted or substituted with one or more halogen atoms,
   ▪ a hydroxy group, in particular a protected hydroxy group, for example a hydroxy group protected with a *tert*-butyldiphenylsilyl ether (tBDPS) protecting group;
   ▪ a (C₁-C₆) alkoxy group, in particular a (C₁-C₄) alkoxy group and more particularly a methoxy group,
   ▪ a -NH₂ group,
   or two R₃, that are borne by the same carbon atom, form with the carbon atom bearing them a (C₃-C₆)cycloalkyl ring, in particular a cyclopropyl ring, unsubstituted or substituted with one or more fluorine atoms, and
- R₄ is chosen from:
   ▪ a hydrogen atom,
   ▪ a (C₁-C₆)alkyl group, in particular a (C₁-C₄)alkyl group and more particularly a methyl group, unsubstituted or substituted with one or more halogen atoms;
or R₃ and R₄, when they are borne by two adjacent carbon atoms, form with the carbon atoms bearing them a (C₃-C₆)cycloalkyl ring, unsubstituted or substituted with one or more halogen atom.

In an embodiment, in the intermediate compounds of formula (I-A) or (II-A):
- m is 0, 1 or 2,
- each R₃, when present, is independently chosen from:
   ▪ a fluorine atom,
   ▪ a (C₁-C₆)alkyl group, in particular a (C₁-C₄)alkyl group and more particularly a methyl,
   ▪ a hydroxy group, in particular a protected hydroxy group, for example a hydroxy group protected with a *tert*-butyldiphenylsilyl ether (tBDPS) protecting group;
   ▪ a (C₁-C₆) alkoxy group, in particular a (C₁-C₄) alkoxy group and more particularly a methoxy group,
   or two R₃, that are borne by the same carbon atom, form with the carbon atom bearing them a (C₃-C₆)cycloalkyl ring, in particular a cyclopropyl ring, substituted with one or more fluorine atoms, and
- R₄ is chosen from:
   ▪ a hydrogen atom, and
   ▪ a (C₁-C₆)alkyl group, in particular a (C₁-C₄)alkyl group and more particularly a methyl group.

In another embodiment, in the intermediate compounds of formula (I-A) or (II-A):
- m is 0 or 1;
- R₃, when present, is chosen from:
   ▪ a hydroxy group, and
   ▪ a (C₁-C₆) alkoxy group, in particular a (C₁-C₄) alkoxy group and more particularly a methoxy group,
   preferably R₃ is a hydroxy group; and
- R₄ is a hydrogen atom.

Among the intermediate compounds of formula (I-A), mention may be made in particular of the following compounds, or a deuterated or tritiated form of the compound of formula (I), or a pharmaceutically acceptable salt thereof:
(21) tert-butyl (S)-2-carbamothioyl-4,4-difluoropyrrolidine-1-carboxylate;
(22) tert-butyl (2S,4R)-2-carbamothioyl-4-fluoropyrrolidine-1-carboxylate,
(23) tert-butyl (2S,4S)-2-carbamothioyl-4-fluoropyrrolidine-1-carboxylate,
(24) tert-butyl (6S)-6-carbamothioyl-1,1-difluoro-5-azaspiro[2.4]heptane-5-carboxylate,
(25) tert-butyl (S)-2-carbamothioylpyrrolidine-1-carboxylate,
(26) tert-butyl (S)-2-carbamothioyl-2-methylpyrrolidine-1-carboxylate,
(27) tert-butyl (2S,4R)-2-carbamothioyl-4-methoxypyrrolidine-1-carboxylate,
(28) tert-butyl (2S,4S)-2-carbamothioyl-4-methoxypyrrolidine-1-carboxylate,
(29) tert-butyl (2S,SR)-2-carbamothioyl-5-methylpyrrolidine-1-carboxylate,
(30) tert-butyl (2S,5S)-2-carbamothioyl-5-methylpyrrolidine-1-carboxylate,
(31) tert-butyl (2S,4S)-2-carbamothioyl-4-methylpyrrolidine-1-carboxylate,
(32) tert-butyl (2S,4S)-2-carbamothioyl-4-(trifluoromethyl)pyrrolidine-1-carboxylate,
(33) tert-butyl (2S,4R)-4-((tert-butyldiphenylsilyl)oxy)-2-carbamothioylpyrrolidine-1-carboxylate,
(34) tert-butyl (2S,3S)-3-((tert-butyldiphenylsilyl)oxy)-2-carbamothioylpyrrolidine-1-carboxylate,
(35) tert-butyl (2S,3S)-2-carbamothioyl-3-methoxypyrrolidine-1-carboxylate,
(36) tert-butyl (S)-6-carbamothioyl-5-azaspiro[2.4]heptane-5-carboxylate, and
(37) tert-butyl (2S,4S)-4-((tert-butyldiphenylsilyl)oxy)-2-carbamothioylpyrrolidine-1-carboxylate.

More particularly, a compound according to the invention may be selected from the group consisting of the following compounds, or a deuterated or tritiated form of the compound of formula (I), or a pharmaceutically acceptable salt thereof:
(21) tert-butyl (S)-2-carbamothioyl-4,4-difluoropyrrolidine-1-carboxylate;
(23) tert-butyl (2S,4S)-2-carbamothioyl-4-fluoropyrrolidine-1-carboxylate,
(24) tert-butyl (6S)-6-carbamothioyl-1,1-difluoro-5-azaspiro[2.4]heptane-5-carboxylate,
(26) tert-butyl (S)-2-carbamothioyl-2-methylpyrrolidine-1-carboxylate,
(28) tert-butyl (2S,4S)-2-carbamothioyl-4-methoxypyrrolidine-1-carboxylate,
(29) tert-butyl (2S,SR)-2-carbamothioyl-5-methylpyrrolidine-1-carboxylate,
(30) tert-butyl (2S,5S)-2-carbamothioyl-5-methylpyrrolidine-1-carboxylate,
(31) tert-butyl (2S,4S)-2-carbamothioyl-4-methylpyrrolidine-1-carboxylate,
(32) tert-butyl (2S,4S)-2-carbamothioyl-4-(trifluoromethyl)pyrrolidine-1-carboxylate,
(34) tert-butyl (2S,3S)-3-((tert-butyldiphenylsilyl)oxy)-2-carbamothioylpyrrolidine-1-carboxylate,
(35) tert-butyl (2S,3S)-2-carbamothioyl-3-methoxypyrrolidine-1-carboxylate,
(36) tert-butyl (S)-6-carbamothioyl-5-azaspiro[2.4]heptane-5-carboxylate, and
(37) tert-butyl (2S,4S)-4-((tert-butyldiphenylsilyl)oxy)-2-carbamothioylpyrrolidine-1-carboxylate.

As for the intermediate compounds of formula (II-B), R₁ and R₂ may have any one of the specific definitions as mentioned above for the compounds of formula (I).

In an embodiment, in the intermediate compounds of formula (II-B):
- R₁ is a (C₁-C₆)alkyl group, in particular a (C₁-C₄)alkyl group and more particularly a *tert*-butyl group, unsubstituted or substituted with one or more fluorine atoms,
- R₂ is chosen from:
   ▪ a hydrogen atom, and
   ▪ a (C₁-C₆)alkyl group, in particular a (C₁-C₄)alkyl group and more particularly a methyl group.

In another embodiment, in the intermediate compounds of formula (III-B):
- R₁ is a (C₁-C₆)alkyl group, in particular a (C₁-C₄)alkyl group and more particularly a *tert*-butyl group, unsubstituted or substituted with one to three fluorine atoms, and
- R₂ is a (C₁-C₆)alkyl group, in particular a (C₁-C₄)alkyl group and more particularly a methyl group.

Intermediate compounds of formula (II-B) may be commercially available. One may mention for example 4-Methyl-5-[2-(2,2,2-trifluoro-1,1-dimethylethyl)-4-pyridinyl]-2-thiazolamine (CAS 1357476-69-7) sold by AURUM pharmatech, 5-[2-(2,2,2-Trifluoro-1,1-dimethylethyl)-4-pyridinyl]-2-thiazolamine (CAS 1395492-61-1) sold by Carbosynth and 5-[2-(1,1-Dimethylethyl)-4-pyridinyl]-2-thiazolamine (CAS 1395492-83-7) sold by Matrix Scientific.

The following tables 1a and 1b comprises respectively specific compounds of formula (I) (basic formula and structure) in accordance with the present disclosure: as well as their characterization (specific optical rotation, ¹H NMR and high-resolution electrospray ionization mass spectrometry HRMS-ESI).:
Optical rotations (aD) were measured on a Perkin Elmer polarimeter (model 341) at 20 °C.
¹H NMR and ¹³C NMR spectra were recorded on Bruker Avance II 500 spectrometer, at 500 MHz (H value) or 125 MHz (C value) with the chemical shifts (δ in ppm) in the solvent dimethyl sulfoxide-d6 (d6-DMSO) referenced at 2.5 ppm at a temperature of 300 K. Coupling constants (1) are reported in Hertz.

High Resolution mass spectra were recorded on a ThermoFischer Exactive Orbitrap spectrometer.

The compounds of the formula (I) can be prepared by the following processes.

Unless otherwise mentioned, R₁, R₂, R₃, R₄ and m are as defined previously.

The compounds of the formula (I) and other related compounds having different substituents are synthesized using techniques and materials described below or otherwise known by the skilled person in the art. In addition, solvents, temperatures, and other reaction conditions presented below may vary as deemed appropriate to the skilled person in the art.

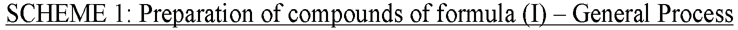

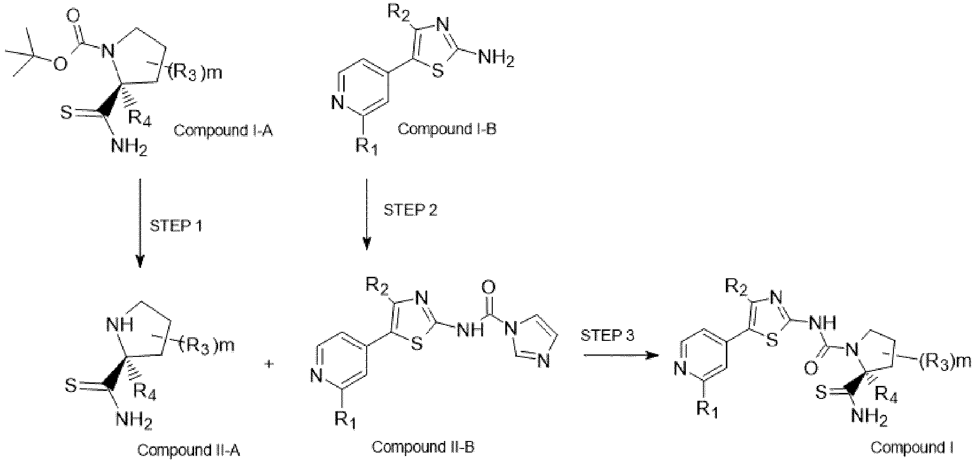

According to SCHEME 1, compound of formula (I) can be obtained in STEP 3 by coupling between compound II-A in which R₄, R₃ and m are as defined above and compound II-B in which R₁ and R₂ are as defined above.

Compound II-A can be obtained from the corresponding compound in which the nitrogen atom is in a protected form, for example from compound I-A having the nitrogen atom protected with a tert-butyl carbamate (Boc) group, by a deprotection STEP 1, for example in an aqueous solution of HCl. More particularly, STEP 1 can be performed by placing compound I-A in a HCl solution with a proper solvent, such as ether or dioxane, and stirred at room temperature (namely at a temperature between 20 and 25 °C) for several hours. The solvent can then be removed in vacuo to obtain compound II-A that is then engaged in the coupling step.

Compound II-B can be obtained from compound I-B in STEP 2 by reaction with 1,1'-carbonyldiimidazole. More particularly, compound I-B can be dissolved in an organic solvent, such as methylene chloride, and 1,1'-carbonyldiimidazole can be added in a molar ratio ranging from 1 to 2, in particular of 1.7. The reaction mixture can be stirred at a temperature ranging between 50 °C and 70 °C, for a duration ranging from 12 to 16 hours, and then cooled to room temperature and filtrated to obtain compound II-B as the precipitate.

Compound II-A and compound II-B are then engaged in the coupling STEP 3 at room temperature using triethylamine (TEA) in polar aprotic organic solvent such as N,N-dimethylformamide (DMF) or acetonitrile. More particularly, compounds II-A and II-B can be dissolved in DMF, for example in a molar proportion of 1.3/1, and TEA can then be added at a molar ratio ranging from 2.5 and 3.5, in particular of 3. The reaction mixture can be stirred at room temperature for a duration of between 12 and 16 hours and treated by an aqueous solvent, such as water. The organic phases are extracted, for example by methylene chloride, washed, in particular by brine, and dried, for instance over sodium sulfate. The solvent can then be evaporated, leading to a crude product that can be further purified, in particular by preparative HPLC using a water/acetonitrile gradient, to obtain compound I.

When R₃ is a hydroxy group, R₃ is protected in compounds I-A and II-A before the coupling step, for example with a tert-butyldiphenylsilyl ether (tBDPS) protecting group. After the coupling step between compound II-A with a protected hydroxy group R₃ and compound II-B, deprotection of the hydroxy group, for example using tetra-n-butylammonium fluoride (TBAF) for a tBDPS protecting group, can be performed on the obtained precipitate and before HPLC purification.

### Intermediate compound I-A can be prepared by a process as shown in SCHEME 2.

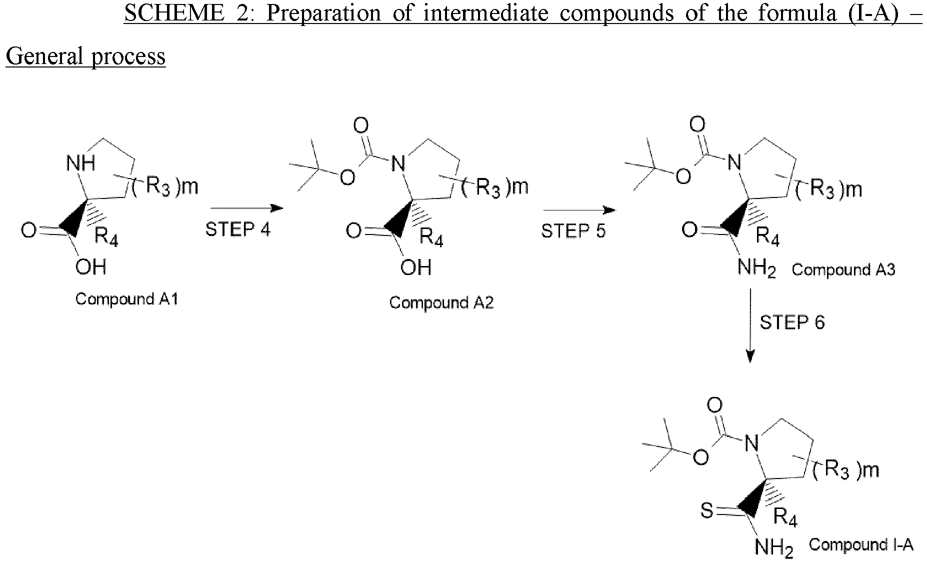

According to SCHEME 2 in which R₃, R₄ and m are as defined above, the nitrogen atom in compound A1 is first protected, for example with a tert-butyl carbamate (Boc) group, in STEP 4. The protection step can be performed by reacting compound A1 with di-tert-butyl dicarbonate in the presence of a base such as triethylamine. For example, compound A1 can be dissolved in an appropriate solvent, such as dichloromethane (DCM). Then, at low temperature, in particular at 0 °C, a base is added, such as triethylamine, in a molar ratio ranging from 1 to 2, for example of 1.5, along with di-tert-butyl dicarbonate, in a molar ratio ranging from 1 to 2, for example of 1.2, and an appropriate nucleophilic catalyst, such as 4-dimethylaminopyridine (DMAP), in a molar ratio ranging from 0.01 to 0.5, for example of 0.15. The reaction mixture can then be stirred at low temperature, such as 0 °C, for a duration of between 15 and 45 minutes, and then at room temperature for a duration of between 8 and 15 hours. The reaction can then be quenched, for example using sodium bicarbonate (NaHCO₃) in an aqueous solution, and extracted, for example using an appropriate solvent, such as dichloromethane. The organic phase can then be dried, for example over Na₂SO₄ anhydrous, filtered and concentrated, for example in vacuo. The crude product can then be purified, for example using flash chromatography, to obtain compound A2.

Compound A2 can be converted in STEP 5 to compound A3 through suitable conditions to transform the carboxylic acid function into the corresponding amide. For example, STEP 5 can be performed by activation with isobutyl chloroformate in the presence of a base, such as triethylamine, followed by reacting with ammonia, preferably an aqueous solution of ammonia. An extraction with an appropriate organic solvent, for example ethyl acetate, followed by filtration and drying, for example over anhydrous magnesium sulphate, and purification for example by flash chromatography, can give compound A3.

Compound A3 can be converted in STEP 6 to compound A4 by converting the amide function into a thioamide, using for example a Lawesson's reagent.

When R₃ is an hydroxy group, R₃ of compound A2 is protected before performing STEP 5, for example with a tert-butyldiphenylsilyl ether (tBDPS) protecting group. In this case, compound I-A with a protected hydroxy group R₃ is obtained, that can be engaged in the coupling reaction as described above in SCHEME 1.

In a particular embodiment, compound A2 in which R₃ is an alkoxy group can be obtained from a compound A2 in which R₃ is a hydroxy group by converting the hydroxy group into an alkoxy group, for example through a reaction with alkyl iodide in tetrahydrofuran, before performing STEP 5.

Herein is also provided a process for preparing a compound of formula (I) as defined above, comprising a coupling reaction between a compound of formula (II-A) and a compound of formula (II-B) 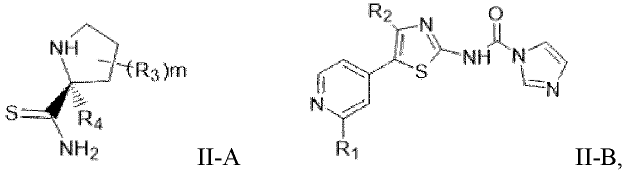
in which R₃, R₄, m, R₂ and R₁ are as defined above, provided that, when R₃ is an hydroxy group, R₃ is protected in compound II-A before the coupling reaction, for example with a *tert*-butyldiphenylsilyl ether (tBDPS) protecting group; the deprotection being performed after the coupling reaction;
wherein said coupling reaction is preferably performed at room temperature using triethylamine (TEA) in polar aprotic organic solvent such as dimethylformamide (DMF).

Said coupling reaction can be optionally preceded by a step for obtaining compound II-A wherein a compound of formula I-A 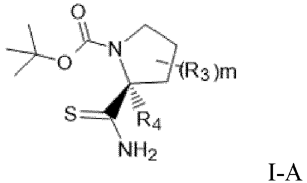 wherein R₃, R₄ and m are as defined above, or R₃ is a protected hydroxy group, for example a hydroxy group protected with a *tert*-butyldiphenylsilyl ether (tBDPS) protecting group, is converted to a compound II-A by a deprotection step, for example by an aqueous solution of HCl.

Said coupling reaction can be optionally preceded by a step for obtaining compound II-B wherein a compound of formula I-B 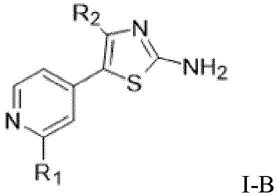 wherein R₁ and R₂ are as defined above, is converted to compound II-B by reaction with 1,1'-carbonyldiimidazole, in particular in an organic solvent, such as methylene chloride.

Herein are further provided the intermediate compounds of formulae (I-A) and (II-A) or, or a deuterated or tritiated form of the compound of formula (I), any of their pharmaceutically acceptable salts: 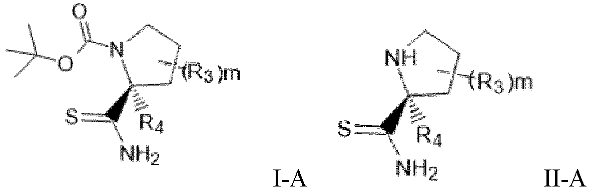 wherein R₃, R₄ and m are as defined above; or R₃ represents a protected hydroxy group, for example a hydroxy group protected with a *tert*-butyldiphenylsilyl ether (tBDPS) protecting group.

In particular, in intermediate compounds of formula (I-A) or (II-A), m, R₃ and R₄ may have any one of the specific definitions as mentioned above for the compounds of formula (I).

In an embodiment, in the intermediate compounds of formula (I-A) or (II-A) :
- m is 0, 1 or 2,
- each R₃, when present, is independently chosen from:
   ▪ a fluorine atom,
   ▪ a (C₁-C₆)alkyl group, in particular a (C₁-C₄)alkyl group and more particularly a methyl group, unsubstituted or substituted with one or more halogen atoms,
   ▪ a (C₃-C₆)cycloalkyl group unsubstituted or substituted with one or more halogen atoms,
   ▪ a hydroxy group, in particular a protected hydroxy group, for example a hydroxy group protected with a *tert*-butyldiphenylsilyl ether (tBDPS) protecting group;
   ▪ a (C₁-C₆) alkoxy group, in particular a (C₁-C₄) alkoxy group and more particularly a methoxy group,
   ▪ a -NH₂ group,
   or two R₃, that are borne by the same carbon atom, form with the carbon atom bearing them a (C₃-C₆)cycloalkyl ring, in particular a cyclopropyl ring, unsubstituted or substituted with one or more fluorine atoms, and
- R₄ is chosen from:
   ▪ a hydrogen atom,
   ▪ a (C₁-C₆)alkyl group, in particular a (C₁-C₄)alkyl group and more particularly a methyl group, unsubstituted or substituted with one or more halogen atoms;
or R₃ and R₄, when they are borne by two adjacent carbon atoms, form with the carbon atoms bearing them a (C₃-C₆)cycloalkyl ring, unsubstituted or substituted with one or more halogen atom.

In an embodiment, in the intermediate compounds of formula (I-A) or (II-A):
- m is 0, 1 or 2,
- each R₃, when present, is independently chosen from:
   ▪ a fluorine atom,
   ▪ a (C₁-C₆)alkyl group, in particular a (C₁-C₄)alkyl group and more particularly a methyl,
   ▪ a hydroxy group, in particular a protected hydroxy group, for example a hydroxy group protected with a *tert*-butyldiphenylsilyl ether (tBDPS) protecting group;
   ▪ a (C₁-C₆) alkoxy group, in particular a (C₁-C₄) alkoxy group and more particularly a methoxy group,
   or two R₃, that are borne by the same carbon atom, form with the carbon atom bearing them a (C₃-C₆)cycloalkyl ring, in particular a cyclopropyl ring, substituted with one or more fluorine atoms, and
- R₄ is chosen from:
   ▪ a hydrogen atom, and
   ▪ a (C₁-C₆)alkyl group, in particular a (C₁-C₄)alkyl group and more particularly a methyl group.

In another embodiment, in the intermediate compounds of formula (I-A) or (II-A):
- m is 0 or 1;
- R₃, when present, is chosen from:
   ▪ a hydroxy group, and
   ▪ a (C₁-C₆) alkoxy group, in particular a (C₁-C₄) alkoxy group and more particularly a methoxy group,
      preferably R₃ is a hydroxy group; and
- R₄ is a hydrogen atom.

Among the intermediate compounds of formula (I-A), mention may be made in particular of the following compounds, or a deuterated or tritiated form of the compound of formula (I), or a pharmaceutically acceptable salt thereof:
(21) tert-butyl (S)-2-carbamothioyl-4,4-difluoropyrrolidine-1-carboxylate;
(22) tert-butyl (2S,4R)-2-carbamothioyl-4-fluoropyrrolidine-1-carboxylate,
(23) tert-butyl (2S,4S)-2-carbamothioyl-4-fluoropyrrolidine-1-carboxylate,
(24) tert-butyl (6S)-6-carbamothioyl-1,1-difluoro-5-azaspiro[2.4]heptane-5-carboxylate,
(25) tert-butyl (S)-2-carbamothioylpyrrolidine-1-carboxylate,
(26) tert-butyl (S)-2-carbamothioyl-2-methylpyrrolidine-1-carboxylate,
(27) tert-butyl (2S,4R)-2-carbamothioyl-4-methoxypyrrolidine-1-carboxylate,
(28) tert-butyl (2S,4S)-2-carbamothioyl-4-methoxypyrrolidine-1-carboxylate,
(29) tert-butyl (2S,SR)-2-carbamothioyl-5-methylpyrrolidine-1-carboxylate,
(30) tert-butyl (2S,5S)-2-carbamothioyl-5-methylpyrrolidine-1-carboxylate,
(31) tert-butyl (2S,4S)-2-carbamothioyl-4-methylpyrrolidine-1-carboxylate,
(32) tert-butyl (2S,4S)-2-carbamothioyl-4-(trifluoromethyl)pyrrolidine-1-carboxylate,
(33) tert-butyl (2S,4R)-4-((tert-butyldiphenylsilyl)oxy)-2-carbamothioylpyrrolidine-1-carboxylate,
(34) tert-butyl (2S,3S)-3-((tert-butyldiphenylsilyl)oxy)-2-carbamothioylpyrrolidine-1-carboxylate,
(35) tert-butyl (2S,3S)-2-carbamothioyl-3-methoxypyrrolidine-1-carboxylate,
(36) tert-butyl (S)-6-carbamothioyl-5-azaspiro[2.4]heptane-5-carboxylate, and
(37) tert-butyl (2S,4S)-4-((tert-butyldiphenylsilyl)oxy)-2-carbamothioylpyrrolidine-1-carboxylate.

More particularly, a compound according to the invention may be selected from the group consisting of the following compounds, or a deuterated or tritiated form of the compound of formula (I), or a pharmaceutically acceptable salt thereof, or deuterium or tritium isotopes thereof:
(21) tert-butyl (S)-2-carbamothioyl-4,4-difluoropyrrolidine-1-carboxylate;
(23) tert-butyl (2S,4S)-2-carbamothioyl-4-fluoropyrrolidine-1-carboxylate,
(24) tert-butyl (6S)-6-carbamothioyl-1,1-difluoro-5-azaspiro[2.4]heptane-5-carboxylate, (26) tert-butyl (S)-2-carbamothioyl-2-methylpyrrolidine-1-carboxylate,
(28) tert-butyl (2S,4S)-2-carbamothioyl-4-methoxypyrrolidine-1-carboxylate,
(29) tert-butyl (2S,SR)-2-carbamothioyl-5-methylpyrrolidine-1-carboxylate,
(30) tert-butyl (2S,5S)-2-carbamothioyl-5-methylpyrrolidine-1-carboxylate,
(31) tert-butyl (2S,4S)-2-carbamothioyl-4-methylpyrrolidine-1-carboxylate,
(32) tert-butyl (2S,4S)-2-carbamothioyl-4-(trifluoromethyl)pyrrolidine-1-carboxylate,
(34) tert-butyl (2S,3S)-3-((tert-butyldiphenylsilyl)oxy)-2-carbamothioylpyrrolidine-1-carboxylate,
(35) tert-butyl (2S,3S)-2-carbamothioyl-3-methoxypyrrolidine-1-carboxylate,
(36) tert-butyl (S)-6-carbamothioyl-5-azaspiro[2.4]heptane-5-carboxylate, and
(37) tert-butyl (2S,4S)-4-((tert-butyldiphenylsilyl)oxy)-2-carbamothioylpyrrolidine-1-carboxylate.

As for the intermediate compounds of formula (II-B), R₁ and R₂ may have any one of the specific definitions as mentioned above for the compounds of formula (I).

In an embodiment, in the intermediate compounds of formula (II-B):
- R₁ is a (C₁-C₆)alkyl group, in particular a (C₁-C₄)alkyl group and more particularly a *tert-*butyl group, unsubstituted or substituted with one or more fluorine atoms,
- R₂ is chosen from:
   ▪ a hydrogen atom, and
   ▪ a (C₁-C₆)alkyl group, in particular a (C₁-C₄)alkyl group and more particularly a methyl group.

In another embodiment, in the intermediate compounds of formula (III-B):
- R₁ is a (C₁-C₆)alkyl group, in particular a (C₁-C₄)alkyl group and more particularly a *tert-*butyl group, unsubstituted or substituted with one to three fluorine atoms, and
- R₂ is a (C₁-C₆)alkyl group, in particular a (C₁-C₄)alkyl group and more particularly a methyl group.

Intermediate compounds of formula (II-B) may be commercially available. One may mention for example 4-Methyl-5-[2-(2,2,2-trifluoro-1,1-dimethylethyl)-4-pyridinyl]-2-thiazolamine (CAS 1357476-69-7) sold by AURUM pharmatech, 5-[2-(2,2,2-Trifluoro-1,1-dimethylethyl)-4-pyridinyl]-2-thiazolamine (CAS 1395492-61-1) sold by Carbosynth and 5-[2-(1,1-Dimethylethyl)-4-pyridinyl]-2-thiazolamine (CAS 1395492-83-7) sold by Matrix Scientific.

The following tables 1a and 1b comprise respectively specific compounds of formula (I) (basic formula and structure) in accordance with the present disclosure as well as their characterization (specific optical rotation, ¹H NMR and high-resolution electrospray ionization mass spectrometry HRMS-ESI).

Optical rotations (αD) were measured on a Perkin Elmer polarimeter (model 341) at 20 °C.

¹H NMR and ¹³C NMR spectra were recorded on Bruker Avance II 500 spectrometer, at 500 MHz (H value) or 125 MHz (C value) with the chemical shifts (δ in ppm) in the solvent dimethyl sulfoxide-d6 (d6-DMSO) referenced at 2.5 ppm at a temperature of 300 K. Coupling constants (1) are reported in Hertz.
High Resolution mass spectra were recorded on a ThermoFischer Exactive Orbitrap spectrometer.

**Table 1a: Compounds (1) to (20).**

| **Cpd N°** | **Basic formula** | **Structure** | **Name** |
|---|---|---|---|
| **1** | C₁₉H₂₀F₅N₅ OS₂ | 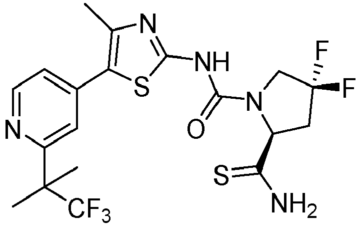 | (S)-2-carbamothioyl-4,4-difluoro-*N*-(4-methyl-5-(2-(1,1,1-trifluoro-2-methylpropan-2-yl)pyridin-4-yl)thiazol-2-yl)pyrrolidine-1-carboxamide |
| **2** | C₁₉H₂₁F₄N₅ OS₂ | 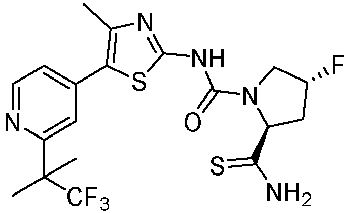 | (2S,4R)-2-carbamothioyl-4-fluoro-*N*-(4-methyl-5-(2-(1,1,1-trifluoro-2-methylpropan-2-yl)pyridin-4-yl)thiazol-2-yl)pyrrolidine-1-carboxamide |
| **3** | C₁₉H₂₁F₄N₅ OS₂ | 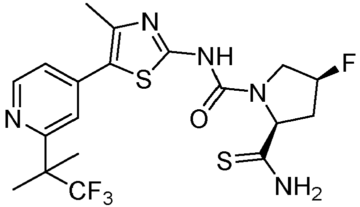 | (2S,4S)-2-carbamothioyl-4-fluoro-*N*-(4-methyl-S-(2-(1,1,1-trifluoro-2-methylpropan-2-yl)pyridin-4-yl)thiazol-2-yl)pyrrolidine-1-carboxamide |
| **4** | C₂₁H₂₂F₅N₅ OS₂ | 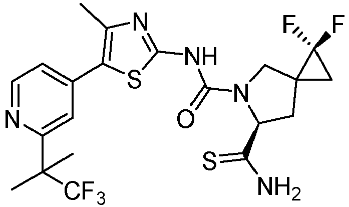 | (S)-6-carbamothioyl-*N*-(4-methyl-5-(2-(1,1,1-trifluoro-2-methylpropan-2-yl)pyridin-4-yl)thiazol-2-yl)-1,1-difluoro-5-azaspiro[2,4]heptane-5-carboxamide |
| **5** | C₁₉H₂₂F₃N₅ OS₂ | 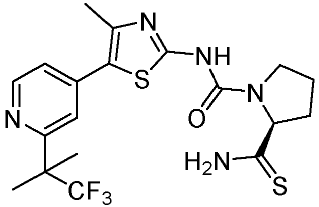 | (S)-2-carbamothioyl-*N-*(4-methyl-5-(2-(1,1,1-trifluoro-2-methylpropan-2-yl)pyridin-4-yl)thiazol-2-yl)pyrrolidine-1-carboxamide |
| **6** | C₂₀H₂₄F₃N₅ OS₂ | 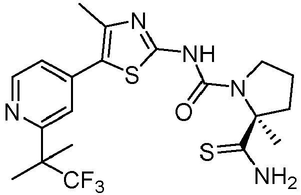 | (S)-2-carbamothioyl-2-methyl-*N-*(4-methyl-5-(2-(1,1,1-trifluoro-2-methylpropan-2-yl)pyridin-4-yl)thiazol-2-yl)pyrrolidine-1-carboxamide |
| **7** | C₂₀H₂₄F₃N₅ O₂S₂ | 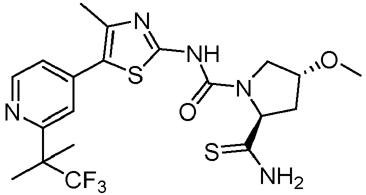 | (2S,4R)-2-carbamothioyl-4-methoxy-*N*-(4-methyl-5-(2-(1,1,1-trifluoro-2-methylpropan-2-yl)pyridin-4-yl)thiazol-2-yl)pyrrolidine-1-carboxamide |
| **8** | C₂₀H₂₄F₃N₅ O₂S₂ | 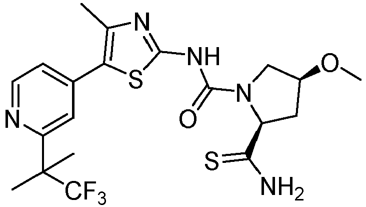 | (2S,4S)-2-carbamothioyl-4-methoxy-*N*-(4-methyl-5-(2-(1,1,1-trifluoro-2-methylpropan-2-yl)pyridin-4-yl)thiazol-2-yl)pyrrolidine-1-carboxamide |
| **9** | C₂₀H₂₄F₃N₅ OS₂ | 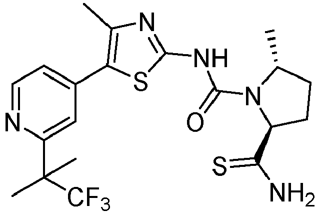 | (2S,5R)-2-carbamothioyl-5-methyl-*N*-(4-methyl-5-(2-(1,1,1-trifluoro-2-methylpropan-2-yl)pyridin-4-yl)thiazol-2-yl)pyrrolidine-1-carboxamide |
| **10** | C₂₀H₂₄F₃N₅ OS₂ | 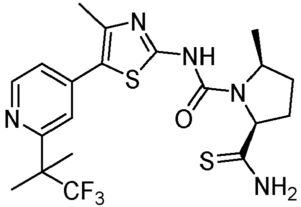 | (2S,5S)-2-carbamothioyl-5-methyl-*N*-(4-methyl-5-(2-(1,1,1-trifluoro-2-methylpropan-2-yl)pyridin-4-yl)thiazol-2-yl)pyrrolidine-1-carboxamide |
| **11** | C₂₀H₂₄F₃N₅ OS₂ | 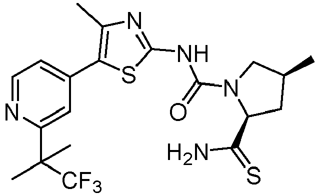 | (2S,4S)-2-carbamothioyl-4-methyl-N-(4-methyl-5-(2-(1,1,1-trifluoro-2-methylpropan-2-yl)pyridin-4-yl)thiazol-2-yl)pyrrolidine-1-carboxamide |
| **12** | C₂₀H₂₁F₆N₅ OS₂ | 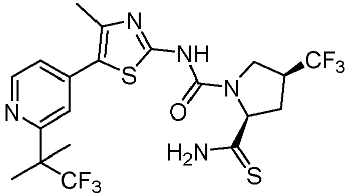 | (2S,4S)-2-carbamothioyl-N-(4-methyl-5-(2-(1,1,1-trifluoro-2-methylpropan-2-yl)pyridin-4-yl)thiazol-2-yl)-4-(trifluoromethyl)pyrrolidine-1-carboxamide |
| **13** | C₁₉H₂₂F₃N₅ O₂S₂ | 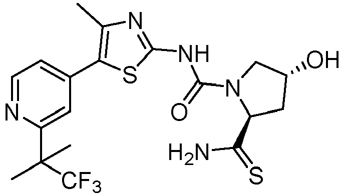 | (2S,4R)-2-carbamothioyl-4-hydroxy-N-(4-methyl-5-(2-(1,1,1-trifluoro-2-methylpropan-2-yl)pyridin-4-yl)thiazol-2-yl)pyrrolidine-1-carboxamide |
| **14** | C₁₉H₂₂F₃N₅ O₂S₂ | 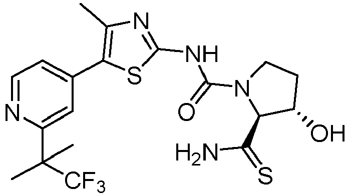 | (2S,3S)-2-carbamothioyl-3-hydroxy-N-(4-methyl-5-(2-(1,1,1-trifluoro-2-methylpropan-2-yl)pyridin-4-yl)thiazol-2-yl)pyrrolidine-1-carboxamide |
| **15** | C₂₀H₂₄F₃N₅ O₂S₂ | 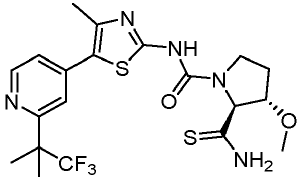 | (2S,3S)-2-carbamothioyl-3-methoxy-N-(4-methyl-5-(2-(1,1,1-trifluoro-2-methylpropan-2-yl)pyridin-4-yl)thiazol-2-yl)pyrrolidine-1-carboxamide |
| **16** | C₂₁H₂₄F₃N₅ OS₂ | 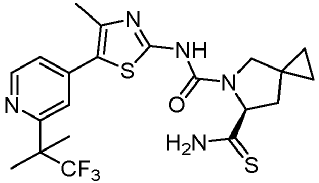 | (S)-6-carbamothioyl-N-(4-methyl-5-(2-(1,1,1-trifluoro-2-methylpropan-2-yl)pyridin-4-yl)thiazol-2-yl)-5-azaspiro[2.4]heptane-5-carboxamide |
| **17** | C₁₉H₂₂F₃N₅ O₂S₂ | 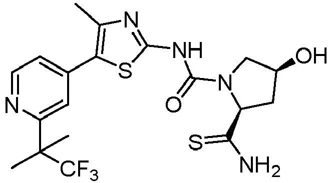 | (2S,4S)-2-carbamothioyl-4-hydroxy-N-(4-methyl-5-(2-(1,1,1-trifluoro-2-methylpropan-2-yl)pyridin-4-yl)thiazol-2-yl)pyrrolidine-1-carboxamide |
| **18** | C₁₉H₂₅N₅O S₂ | 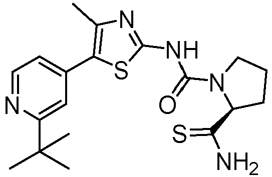 | (S)-2-carbamothioyl-*N*-(4-methyl-5-(2-tert-butyl-pyridin-4-yl)thiazol-2-yl)pyrrolidine-1-carboxamide |
| **19** | C₁₉H₂₅FN₅ OS₂ | 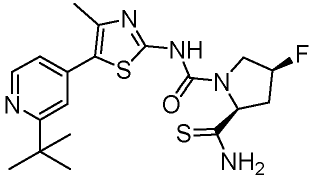 | (2S,4S)-2-carbamothioyl-4-fluoro-*N*-(4-methyl-5-(2-tert-butyl-pyridin-4-yl)thiazol-2-yl)pyrrolidine-1-carboxamide |
| **20** | C₂₁H₂₇N₅O S₂ | 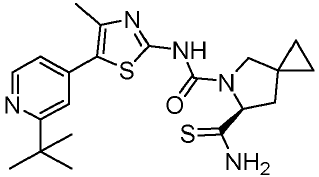 | (S)-6-carbamothioyl-N-(4-methyl-5-(2-tert-butyl-pyridin-4-yl)thiazol-2-yl)-5-azaspiro[2.4]heptane-5-carboxamide |

**Table 1b: Characterization of compounds (1) to (20)**

| **Cpd N°** | **Yield** | **Characterization** |
|---|---|---|
| **1** | 72% | [α]_{D} +8.4 (*c* 0.095, CHCl₃). **¹H NMR** (500 MHz, DMSO) δ 9.81 (s, 1H), 9.40 (s, 1H), 8.61 (d, *J* = 5.2 Hz, 1H), 7.55 (s, 1H), 7.42 (dd, *J* = 5.1, 1.3 Hz, 1H), 4.89 (s, 1H), 4.07 (d, *J* = 10.9 Hz, 2H), 3.07 - 2.89 (m, 1H), 2.52 (d, *J* = 3.2 Hz, 1H), 2.44 - 2.33 (m, 3H), 1.62 (s, 7H). **HRMS** ESI⁺ calculated 494.1102 for C₁₉H₂₁F₅N₅OS₂, found 494.1101 |
| **2** | 58% | [α]_{D} -63.5 (*c* 0.085, CHCl₃). **¹H NMR** (500 MHz, DMSO) δ 11.22 (s, 1H), 9.65 (s, 1H), 9.40 (s, 1H), 8.60 (d, *J* = 5.1 Hz, 1H), 7.56 (s, 1H), 7.42 (dd, *J* = 5.1, 1.5 Hz, 1H), 5.40 (d, *J* = 53.1 Hz, 1H), 4.88 - 4.65 (m, 1H), 4.00 (dt, *J* = 32.9, 16.3 Hz, 1H), 3.75 (dd, *J* = 38.0, 11.7 Hz, 1H), 2.62 - 2.51 (m, 1H), 2.41 (s, 3H), 2.20 - 2.02 (m, 1H), 1.61 (s, 6H). |
| | | **HRMS** ESI⁺ calculated 476.1196 for C₁₉H₂₂F₄N₅OS₂, found 476.1195 |
| **3** | 61% | [α]_{D} +74 (*c* 0.1, CHCl₃). **¹H NMR** (500 MHz, DMSO) δ 11.10 (s, 1H), 9.70 (s, 1H), 9.20 (s, 1H), 8.61 (d, *J* = 5.1 Hz, 1H), 7.55 (s, 1H), 7.42 (dd, *J* = 5.1, 1.2 Hz, 1H), 5.31 (d, *J* = 53.1 Hz, 1H), 4.79 (d, *J* = 7.3 Hz, 1H), 3.97 (s, 1H), 3.78 (ddd, *J* = 36.8, 12.5, 3.9 Hz, 1H), 2.68 - 2.51 (m, 2H), 2.42 (s, 3H), 1.61 (s, 6H). **HRMS** ESI⁺ calculated 476.1196 for C₁₉H₂₂F₄N₅OS₂, found 476.1192 |
| **4** | 59% | [α]_{D} -93.3 (*c* 0.075, CHCl₃). **¹H NMR** (500 MHz, DMSO) δ 11.13 (s, 1H), 9.71 (s, 1H), 9.32 (s, 1H), 8.61 (d, *J* = 5.1 Hz, 1H), 7.55 (s, 1H), 7.42 (d, *J* = 4.2 Hz, 1H), 4.81 (m, 1H), 3.96 - 3.78 (m, 1H), 3.78 - 3.61 (m, 1H), 2.63 (dt, *J* = 17.0, 7.6 Hz, 1H), 2.40 (s, 3H), 2.36 (dt, *J* = 3.5, 1.8 Hz, 1H), 1.99 (d, *J* = 12.3 Hz, 1H), 1.61 (s, 6H), 1.55 (m,1H). |
| | | **HRMS** ESI⁺ calculated 520.1259 for C₂₁H₂₃F₅N₅OS₂, found 520.1253 |
| **5** | 73% | [α]_{D} -144 (*c* 0.125, CHCl₃). **¹H NMR** (500 MHz, DMSO) δ 10.96 (s, 1H), 9.60 (s, 1H), 9.19 (s, 1H), 8.60 (d, *J* = 5.2 Hz, 1H), 7.55 (s, 1H), 7.41 (dd, *J* = 5.2, 1.4 Hz, 1H), 4.67 (s, 1H), 3.71 (s, 1H), 3.54 - 3.43 (m, 1H), 2.41 (s, 3H), 2.27 - 2.15 (m, 1H), 1.89 (m, 3H), 1.61 (s, 6H). **HRMS** ESI⁺ calculated 458.1291 for C₁₉H₂₃F₃N₅OS₂, found 458.1270 |
| **6** | 58% | [α]_{D} +26 (*c* 0.1, CHCl₃). **¹H NMR** (500 MHz, DMSO) δ 10.21 (s, 1H), 8.67 (dd, *J* = 5.1, 0.4 Hz, 1H), 7.64 (s, 1H), 7.50 (dd, *J* = 5.1, 1.6 Hz, 1H), 3.63 (dt, *J* = 11.0, 8.3 Hz, 1H), 3.38 - 3.29 (m, 1H), 2.49 (s, 3H), 2.47 - 2.30 (m, 1H), 2.22 - 2.02 (m, 2H), 2.00 - 1.85 (m, 2H), 1.63 (s, 6H), 1.46 (s, 3H). **HRMS** ESI⁺ calculated 472.1447 for C₂₀H₂₅F₃N₅OS₂, found 472.1818 |
| **7** | 55% | [α]_{D} -37 (*c* 0.075, CHCl₃). **¹H NMR** (500 MHz, DMSO) δ 11.07 (s, 1H), 9.59 (s, 1H), 9.31 (s, 1H), 8.60 (d, *J* = 5.1 Hz, 1H), 7.55 (s, 1H), 7.42 (dd, *J* = 5.1, 1.3 Hz, 1H), 4.67 (d, *J* = 6.0 Hz, 1H), 4.04 (s, 1H), 3.76 (d, *J* = 11.0 Hz, 1H), 3.67 (d, *J* = 8.0 Hz, 1H), 3.22 (s, 3H), 2.41 (s, 3H), 2.33 (s, 1H), 1.98 (s, 1H), 1.61 (s, 6H). **HRMS** ESI⁺ calculated 488.1396 for C₂₀H₂₅F₃N₅O₂S₂, found 488.1392 |
| **8** | 65% | [α]_{D} +55 (*c* 0.1, CHCl₃). **¹H NMR** (500 MHz, DMSO) δ 11.00 (s, 1H), 9.59 (s, 1H), 9.07 (s, 1H), 8.60 (d, *J* = 5.1 Hz, 1H), 7.55 (s, 1H), 7.41 (dd, *J* = 5.1, 1.3 Hz, 1H), 4.66 (s, 1H), 3.97 (s, 1H), 3.74 (dd, *J* = 11.0, 5.2 Hz, 1H), 3.67 (t, *J* = 8.4 Hz, 1H), 3.18 (s, 3H), 2.44-2.30 (m, 1H), 2.42 (s, 3H), 2.26 (s, 1H), 1.61 (s, 6H). **HRMS** ESI⁺ calculated 488.1396 for C₂₀H₂₅F₃N₅O₂S₂, found 488.1391 |
| **9** | 51% | [α]_{D} -92.8 (*c* 0.07, CHCl₃). **¹H NMR** (500 MHz, DMSO) δ 10.16 (s, 1H), 8.60 (d, *J* = 5.2 Hz, 1H), 7.55 (s, 1H), 7.42 (dd, *J* = 5.1, 1.4 Hz, 1H), 4.61 - 4.46 (m, 1H), 3.95 (dd, *J* = 14.2, 6.9 Hz, 1H), 2.39 (m, 4H), 2.34 - 2.15 (m, 2H), 1.88 - 1.74 (m, 2H), 1.74 - 1.66 (m, 1H), 1.62 (s, 6H), 1.13 (d, *J* = 5.9 Hz, 3H). **HRMS** ESI⁺ calculated 472.1447 for C₂₀H₂₅F₃N₅OS₂, found 472.1443 |
| **10** | 66% | [α]_{D} -37 (*c* 0.135, CHCl₃). **¹H NMR** (500 MHz, DMSO) δ 9.60 (s, 1H), 9.02 (s, 1H), 8.60 (d, *J* = 5.2 Hz, 1H), 7.54 (s, 1H), 7.41 (dd, *J* = 5.2, 1.5 Hz, 1H), 4.63 (d, *J* = 6.8 Hz, 1H), 4.22 (s, 1H), 2.40 (s, 3H), 2.29 - 2.13 (m, 1H), 2.13 - 1.92 (m, 2H), 1.66 - 1.55 (m, 8H), 1.30 (d, *J* = 6.3 Hz, 3H). **HRMS** ESI⁺ calculated 472.1447 for C₂₀H₂₅F₃N₅OS₂, found 472.1434 |
| **11** | 60% | [α]_{D} -32.7 (*c* 0.165, MeOH). **¹H NMR** (500 MHz, DMSO) δ 10.96 (s, 1H), 9.52 (s, 1H), 9.24 (s, 1H), 8.59 (d, *J* = 5.2 Hz, 2H), 7.54 (s, 2H), 7.41 (dd, *J* = 5.1, 1.3 Hz, 2H), 4.63 (s, 1H), 3.87 (t, *J* = 8.0 Hz, 2H), 3.10 (t, *J* = 9.9 Hz, 1H), 2.41 (s, 6H), 2.22 (d, *J* = 6.2 Hz, 2H), 1.61 (s, 10H), 1.48 (dd, *J* = 20.6, 11.2 Hz, 2H), 1.00 (d, *J* = 6.5 Hz, 5H). **HRMS** ESI⁺ calculated 472.1447 for C₂₀H₂₅F₃N₅OS₂, found 472.1447 |
| **12** | 49% | [α]_{D} 0 (*c* 0.11, CHCl₃). **¹H NMR** (500 MHz, DMSO) δ 11.20 (s, 1H), 9.66 (s, 1H), 9.37 (s, 1H), 8.60 (d, *J* = 5.1 Hz, 1H), 7.55 (s, 1H), 7.42 (dd, *J* = 5.1, 1.2 Hz, 1H), 4.77 (s, 1H), 4.09 (t, *J* = 8.2 Hz, 1H), 3.59 (s, 1H), 3.39 (m, 1H), 2.69 - 2.55 (m, 1H), 2.41 (s, 3H), 2.02 - 1.87 (m, 1H), 1.61 (s, 6H). **HRMS** ESI⁺ calculated 526.1164 for C₂₀H₂₂F₆N₅OS₂, found 526.1161 |
| **13** | 29% | [α]_{D} -61 (*c* 0.1, CHCl₃). **¹H NMR** (500 MHz, DMSO) δ 11.04 (s, 1H), 9.54 (s, 1H), 9.28 (s, 1H), 8.60 (d, *J* = 5.2 Hz, 1H), 7.55 (s, 1H), 7.42 (d, *J* = 4.2 Hz, 1H), 5.14 (s, 1H), 4.72 (s, 1H), 4.33 (s, 1H), 3.67 (d, *J* = 7.4 Hz, 1H), 3.53 (d, *J* = 10.5 Hz, 1H), 2.41 (s, 3H), 2.17 (m, 1H), 1.93 (m, 1H), 1.61 (s, 6H).. **HRMS** ESI⁻ calculated 472.1094 for C₁₉H₂₁F₃N₅O₂S₂, found 472.1101 |
| **14** | 36% | [α]_{D} 0 (*c* 0.1, CHCl₃). **¹H NMR** (500 MHz, DMSO) δ 11.02 (s, 1H), 9.70 (s, 1H), 9.21 (s, 1H), 8.60 (d, *J* = 5.1 Hz, 1H), 7.55 (s, 1H), 7.42 (d, *J* = 4.3 Hz, 1H), 5.46 (s, 1H), 4.52 (s, 1H), 4.28 (s, 1H), 3.79 (s, 1H), 3.58 (dd, *J* = 17.0, 9.7 Hz, 1H), 2.42 (s, 3H), 1.96 (m, 1H), 1.80 (s, 1H), 1.61 (s, 6H). **HRMS** ESI⁻ calculated 472.1094 for C₁₉H₂₁F₃N₅O₂S₂, found 472.1100 |
| **15** | 76% | [α]_{D} -128.6(*c* 0.105, CHCl₃). **¹H NMR** (500 MHz, DMSO) δ 11.07 (s, 1H), 9.79 (s, 1H), 9.34 (s, 1H), 8.61 (d, *J* = 5.2 Hz, 1H), 7.56 (s, 1H), 7.43 (d, *J* = 4.4 Hz, 1H), 4.69 (m, 1H), 3.93 (m, 1H), 3.84 (m, 1H), 3.48 (q, *J* = 9.0 Hz, 1H), 3.33 (s, 3H), 2.43 (s, 3H), 2.01 (m, 2H), 1.62 (s, 6H). **HRMS** ESI⁺ calculated 488.1396 for C₂₀H₂₄F₃N₅O₂S₂, found 488.1394 |
| **16** | 46% | [α]_{D} -9.1 (*c* 0.11, CHCl₃). ¹H NMR (500 MHz, DMSO) δ 10.93 (s, 1H), 9.66 (s, 1H), 9.21 (s, 1H), 8.60 (d, *J* = 5.1 Hz, 1H), 7.55 (s, 1H), 7.41 (dd, *J* = 5.2, 1.4 Hz, 1H), 4.78 (m, 1H), 3.54 (m, 2H), 2.39 (m, 4H), 1.88 - 1.77 (m, 1H), 1.61 (s, 6H), 0.77 - 0.31 (m, 4H). **HRMS** ESI⁺ calculated 484.1447 for C₂₁H₂₅F₃N₅OS₂, found 484.1445 |
| **17** | 40% | [α]_{D} -54.8 (*c* 0.135, CHCl₃). ¹H NMR (500 MHz, DMSO) δ 10.98 (s, 1H), 9.57 (s, 1H), 9.12 (s, 1H), 8.63 - 8.53 (m, 1H), 7.54 (s, 1H), 7.41 (dd, *J* = 5.2, 1.6 Hz, 1H), 5.10 (s, 1H), 4.64 (m, 1H), 4.24 (s, 1H), 3.75 (dd, *J* = 10.5, 5.6 Hz, 1H), 3.55 - 3.43 (m, 1H), 2.41 (m, 4H), 2.03 - 1.90 (m, 1H), 1.61 (s, 6H). **HRMS** ESI⁻ calculated 472.1094 for C₁₉H₂₁F₃N₅O₂S₂, found 472.1103 |
| **18** | 53% | [α]_{D} -36 (*c* 0.1, CHCl₃). **¹H NMR** (500 MHz, DMSO) δ 10.94 (s, 1H), 9.60 (s, 1H), 9.19 (s, 1H), 8.51 (m, 1H), 7.36 (d, *J* = 0.8 Hz, 1H), 7.23 (dd, *J* = 5.2, 1.6 Hz, 1H), 4.67 (s, 1H), 3.70 (s, 1H), 3.48 (m, 1H), 2.41 (s, 3H), 2.25 - 2.15 (m, 1H), 1.89 (m, 3H), 1.34 (s, 9H). **HRMS** ESI⁺ calcd 404.1573 for C₁₉H₂₆N₅OS₂, found 404.1571 |
| **19** | 70% | [α]_{D} +32 (*c* 0.155, MeOH). **¹H NMR** (500 MHz, DMSO) δ 11.05 (s, 1H), 9.69 (s, 1H), 9.19 (s, 1H), 8.52 (dd, *J* = 5.1, 0.5 Hz, 1H), 7.36 (s, 1H), 7.24 (dd, *J* = 5.1, 1.5 Hz, 1H), 5.25 (dd, *J* = 55.3, 28.5 Hz, 1H), 4.78 (d, *J* = 8.6 Hz, 1H), 3.95 (m, 1H), 3.77 (ddd, *J* = 36.8, 12.5, 4.0 Hz, 1H), 2.69 - 2.50 (m, 2H), 2.37 (s, 3H), 1.33 (s, 9H). **HRMS** ESI⁺ calcd 422.1479 for C₁₉H₂₅FN₅OS₂, found 422.1490 |
| **20** | 68% | [α]_{D} +13.6 (*c* 0.22, MeOH). **¹H NMR** (500 MHz, DMSO) δ 10.89 (s, 1H), 9.66 (s, 1H), 9.21 (s, 1H), 8.52 (d, *J* = 5.2 Hz, 1H), 7.35 (m, 1H), 7.24 (dd, *J* = 5.1, 1.5 Hz, 1H), 4.74 (m, 1H), 3.66 - 3.44 (m, 2H), 2.39 (m,4H), 1.88 - 1.77 (m, 1H), 1.35 (s, 9H), 0.68 - 0.39 (m, 4H). **HRMS** ESI⁺ calcd 430.1730 for C₂₁H₂₈N₅OS₂, found 430.1720 |

The following tables 2a and 2b comprises respectively specific intermediate compounds of formula (I-A) (basic formula and structure) in accordance with the present disclosure as well as their characterization (specific optical rotation, ¹H NMR, ¹³C NMR and high-resolution electrospray ionization mass spectrometry HRMS-ESI).

Optical rotations (aD) were measured on a Perkin Elmer polarimeter (model 341) at 20 °C.

¹H NMR and ¹³C NMR spectra were recorded on Bruker Avance II 500 spectrometer, at 500 MHz (H value) or 125 MHz (C value) with the chemical shifts (δ in ppm) in the solvent dimethyl sulfoxide-d6 (d6-DMSO) referenced at 2.5 ppm at a temperature of 300 K. Coupling constants (1) are reported in Hertz.

High Resolution mass spectra were recorded on a ThermoFischer Exactive Orbitrap spectrometer.

**Table 2a: Intermediate compounds (21) to (37).**

| **Cpd N°** | **Basic formula** | **Structure** | **Name** |
|---|---|---|---|
| **21** | C₁₀H₁₆F₂N₂O₂S | 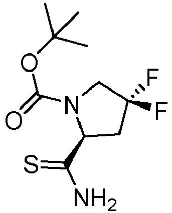 | tert-butyl (S)-2-carbamothioyl-4,4-difluoropyrrolidine-1 - carboxylate |
| **22** | C₁₀H₁₇FN₂O₂S | 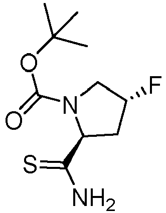 | tert-butyl (2S,4R)-2-carbamothioyl-4-fluoropyrrolidine-1-carboxylate |
| **23** | C₁₁H₁₈FN₂O₂S | 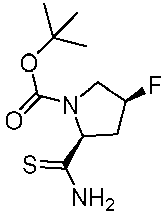 | tert-butyl (2S,4S)-2-carbamothioyl-4-fluoropyrrolidine-1-carboxylate |
| **24** | C₁₂H₁₈F₂N₂O₂S | 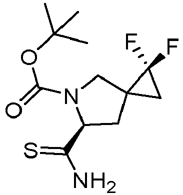 | tert-butyl (6S)-6-carbamothioyl-1,1-difluoro-5-azaspiro[2.4]heptane-5-carboxylate |
| **25** | C₁₀H₁₈N₂O₂S | 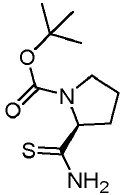 | tert-butyl (S)-2-carbamothioylpyrrolidine-1 - carboxylate |
| **26** | C₁₁H₂₀N₂O₂S | 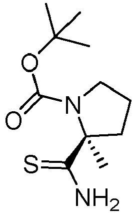 | tert-butyl (S)-2-carbamothioyl-2-methylpyrrolidine-1-carboxylate |
| **27** | C₁₁H₂₀N₂O₃S | 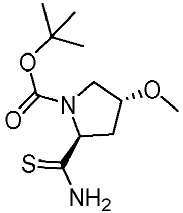 | tert-butyl (2S,4R)-2-carbamothioyl-4-methoxypyrrolidine-1-carboxylate |
| **28** | C₁₁H₂₀N₂O₃S | 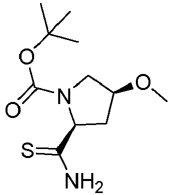 | tert-butyl (2S,4S)-2-carbamothioyl-4-methoxypyrrolidine-1-carboxylate |
| **29** | C₁₁H₂₀N₂O₂S | 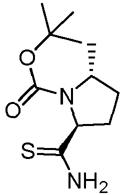 | tert-butyl (2S,5R)-2-carbamothioyl-5-methylpyrrolidine-1-carboxylate |
| **30** | C₁₁H₂₀N₂O₂S | 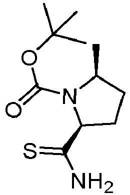 | tert-butyl (2S,5S)-2-carbamothioyl-5-methylpyrrolidine-1-carboxylate |
| **31** | C₁₁H₂₀N₂O₂S | 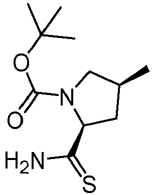 | tert-butyl (2S,4S)-2-carbamothioyl-4-methylpyrrolidine-1-carboxylate |
| **32** | C₁₁H₁₇F₃N₂O₂S | 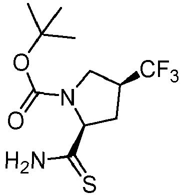 | tert-butyl (2S,4S)-2-carbamothioyl-4-(trifluoromethyl)pyrrolidine-1-carboxylate |
| **33** | C₂₆H₃₆N₂O₃SSi | 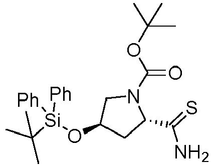 | tert-butyl (2S,4R)-4-((tert-butyldiphenylsilyl)oxy)-2-carbamothioylpyrrolidine-1-carboxylate |
| **34** | C₂₆H₃₆N₂O₃SSi | 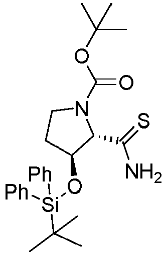 | tert-butyl (2S,3S)-3-((tert-butyldiphenylsilyl)oxy)-2-carbamothioylpyrrolidine-1- carboxylate |
| **35** | C₁₁H₂₀N₂O₃S | 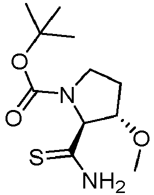 | tert-butyl (2S,3S)-2-carbamothioyl-3-methoxypyrrolidine-1-carboxylate |
| **36** | C₁₂H₂₀N₂O₂S | 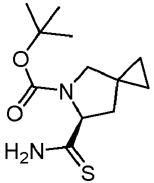 | tert-butyl (S)-6-carbamothioyl-5-azaspiro[2.4]heptane-5-carboxylate |
| **37** | C₂₆H₃₆N₂O₃SSi | 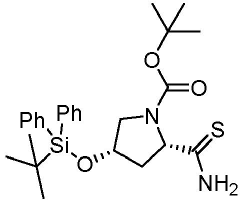 | tert-butyl (2S,4S)-4-((tert-butyldiphenylsilyl)oxy)-2-carbamothioylpyrrolidine-1- carboxylate |

In some embodiments, the PIK3CA inhibitor is an antibody. As used herein, the term "antibody" is used in the broadest sense and specifically covers monoclonal antibodies, polyclonal antibodies, multispecific antibodies (e.g. bispecific antibodies) formed from at least two intact antibodies, and antibody fragments so long as they exhibit the desired biological activity. The term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); BiTE (Bispecific T-cell Engager, scFv-scFv tandems to attract T cells); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affinity ReTargeting"); small antibody mimetics comprising one or more CDRs and the like. The techniques for preparing and using various antibody-based constructs and fragments are well known in the art (see Kabat et al., 1991, specifically incorporated herein by reference). Diabodies, in particular, are further described in EP 404, 097 and WO 93/1 1 161; whereas linear antibodies are further described in Zapata et al. (1995). Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art. For example, each of Beckman et al., 2006; Holliger & Hudson, 2005; Le Gall et al., 2004; Reff & Heard, 2001 ; Reiter et al., 1996; and Young et al., 1995 further describe and enable the production of effective antibody fragments. In some embodiments, the antibody is a "chimeric" antibody as described in U.S. Pat. No. 4,816,567. In some embodiments, the antibody is a humanized antibody, such as described U.S. Pat. Nos. 6,982,321 and 7,087,409. In some embodiments, the antibody is a human antibody. A "human antibody" such as described in US 6,075,181 and 6,150,584. In some embodiments, the antibody is a single domain antibody such as described in EP 0 368 684, WO 06/030220 and WO 06/003388. In a particular embodiment, the inhibitor is a monoclonal antibody. Monoclonal antibodies can be prepared and isolated using any technique that provides for the production of antibody molecules by continuous cell lines in culture. Techniques for production and isolation include but are not limited to the hybridoma technique, the human B-cell hybridoma technique and the EBV-hybridoma technique.

In a particular, the PIK3CA inhibitor is an intrabody having specificity for PI3K. As used herein, the term "intrabody" generally refer to an intracellular antibody or antibody fragment. Antibodies, in particular single chain variable antibody fragments (scFv), can be modified for intracellular localization. Such modification may entail for example, the fusion to a stable intracellular protein, such as, e.g., maltose binding protein, or the addition of intracellular trafficking/localization peptide sequences, such as, e.g., the endoplasmic reticulum retention. In some embodiments, the intrabody is a single domain antibody. In some embodiments, the antibody according to the invention is a single domain antibody. The term "single domain antibody" (sdAb) or "VHH" refers to the single heavy chain variable domain of antibodies of the type that can be found in Camelid mammals which are naturally devoid of light chains. Such VHH are also called "nanobody^{®}". According to the invention, sdAb can particularly be llama sdAb.

In some embodiments, the PIK3CA inhibitor is a short hairpin RNA (shRNA), a small interfering RNA (siRNA) or an antisense oligonucleotide which inhibits the expression of USP14. In a particular embodiment, the inhibitor of USP14 expression is siRNA. A short hairpin RNA (shRNA) is a sequence of RNA that makes a tight hairpin turn that can be used to silence gene expression via RNA interference. shRNA is generally expressed using a vector introduced into cells, wherein the vector utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs that match the siRNA to which it is bound. Small interfering RNA (siRNA), sometimes known as short interfering RNA or silencing RNA, are a class of 20-25 nucleotide-long double- stranded RNA molecules that play a variety of roles in biology. Most notably, siRNA is involved in the RNA interference (RNAi) pathway whereby the siRNA interferes with the expression of a specific gene. Anti-sense oligonucleotides include anti-sense RNA molecules and anti-sense DNA molecules, would act to directly block the translation of the targeted mRNA by binding thereto and thus preventing protein translation or increasing mRNA degradation, thus decreasing the level of the targeted protein, and thus activity, in a cell. For example, antisense oligonucleotides of at least about 15 bases and complementary to unique regions of the mRNA transcript sequence can be synthesized, e.g., by conventional phosphodiester techniques. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732). Antisense oligonucleotides, siRNAs, shRNAs of the invention may be delivered in vivo alone or in association with a vector. In its broadest sense, a "vector" is any vehicle capable of facilitating the transfer of the antisense oligonucleotide, siRNA, shRNA or ribozyme nucleic acid to the cells and typically mast cells. Typically, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

In some embodiments, the inhibitor of PIK3CA expression is an endonuclease. In the last few years, staggering advances in sequencing technologies have provided an unprecedentedly detailed overview of the multiple genetic aberrations in cancer. By considerably expanding the list of new potential oncogenes and tumor suppressor genes, these new data strongly emphasize the need of fast and reliable strategies to characterize the normal and pathological function of these genes and assess their role, in particular as driving factors during oncogenesis. As an alternative to more conventional approaches, such as cDNA overexpression or downregulation by RNA interference, the new technologies provide the means to recreate the actual mutations observed in cancer through direct manipulation of the genome. Indeed, natural and engineered nuclease enzymes have attracted considerable attention in the recent years. The mechanism behind endonuclease-based genome inactivating generally requires a first step of DNA single or double strand break, which can then trigger two distinct cellular mechanisms for DNA repair, which can be exploited for DNA inactivating: the errorprone nonhomologous end-joining (NHEJ) and the high-fidelity homology-directed repair (HDR).

In a particular embodiment, the endonuclease is CRISPR-cas. As used herein, the term "CRISPR-cas" has its general meaning in the art and refers to clustered regularly interspaced short palindromic repeats associated which are the segments of prokaryotic DNA containing short repetitions of base sequences.

In some embodiment, the endonuclease is CRISPR-cas9 which is from Streptococcus pyogenes. The CRISPR/Cas9 system has been described in US 8697359 B1 and US 2014/0068797. Originally an adaptive immune system in prokaryotes (Barrangou and Marraffini, 2014), CRISPR has been recently engineered into a new powerful tool for genome editing. It has already been successfully used to target important genes in many cell lines and organisms, including human (Mali et al., 2013, Science, Vol. 339 : 823-826), bacteria (Fabre et al., 2014, PLoS Negl. Trop. Dis., Vol. 8:e2671.), zebrafish (Hwang et al., 2013, PLoS One, Vol. 8:e68708.), C. elegans (Hai et al., 2014 Cell Res. doi: 10.1038/cr.2014.11.), bacteria (Fabre et al., 2014, PLoS Negl. Trop. Dis., Vol. 8:e2671.), plants (Mali et al., 2013, Science, Vol. 339 : 823-826), Xenopus tropicalis (Guo et al., 2014, Development, Vol. 141 : 707-714.), yeast (DiCarlo et al., 2013, Nucleic Acids Res., Vol. 41 : 4336-4343.), Drosophila (Gratz et al., 2014 Genetics, doi:10.1534/genetics.113.160713), monkeys (Niu et al., 2014, Cell, Vol. 156 : 836-843.), rabbits (Yang et al., 2014, J. Mol. Cell Biol., Vol. 6 : 97-99.), pigs (Hai et al., 2014, Cell Res. doi: 10.1038/cr.2014.11.), rats (Ma et al., 2014, Cell Res., Vol. 24 : 122-125.) and mice (Mashiko et al., 2014, Dev. Growth Differ. Vol. 56 : 122-129.). Several groups have now taken advantage of this method to introduce single point mutations (deletions or insertions) in a particular target gene, via a single gRNA. Using a pair of gRNA-directed Cas9 nucleases instead, it is also possible to induce large deletions or genomic rearrangements, such as inversions or translocations. A recent exciting development is the use of the dCas9 version of the CRISPR/Cas9 system to target protein domains for transcriptional regulation, epigenetic modification, and microscopic visualization of specific genome loci.

In some embodiment, the endonuclease is CRISPR-Cpf1 which is the more recently characterized CRISPR from Provotella and Francisella 1 (Cpf1) in Zetsche et al. ("Cpf1 is a Single RNA-guided Endonuclease of a Class 2 CRISPR-Cas System (2015); Cell; 163, 1-13).

In **a second aspect,** the invention relates to the PIK3CA inhibitor for use according to the invention, and a classical treatment as a combined preparation for simultaneous, separate or sequential use in the treatment of uterine disease in a subject in need thereof

As used herein, the terms **"combined treatment",** "combined therapy" or "therapy combination" refer to a treatment that uses more than one medication. The combined therapy may be dual therapy or bi-therapy.

As used herein, the term **"classical treatment"** refers to treatments well known in the art and used to treat uterine disease (Vannuccini et al 2018, https://doi.org/10.1016/j.fertnstert.2018.01.013).

In the context of the invention, the classical treatment is selected from the group consisting of but not limited to: gonadotropin releasing hormone analogues (GnRH analogues), Progestins (norethindrone acetate (NETA)), Levonorgestrel-releasing intrauterine system (LNG-IUS); combined oral contraceptives, nonsteroidal anti-inflammatory drugs, Aromatase Inhibitors (letrozole [Femara], anastrozole [Arimidex], fadrozole, Selective Progesterone Receptor Modulators, chlormadinone acetate, cyproterone acetate, danazol, desogestrel, drospirenone, dienogest, 5α-dihydroprogesterone, ethanediol acetate, ethynodiol diacetate, etonogestrel, gestodene, estretol, 17-hydroxyprogesterone, levomefolate, levonorgestrel, medroxyprogesterone acetate (17α-hydroxy-6α-methylprogesterone acetate), megestrol, megestrol acetate (17αacetoxy-6-dehydro-6-methylprogesterone), nestorone, nomegestrol acetate, norethindrone, norethindrone acetate, norethynodrel, norgestimate, norgestrel, progesterone, tanaproget, trimegestone, danazol, leuprolide, leuprolide acetate, goserelin, goserelin acetate, histrelin, histrelin acetate, triptorelin, nafarelin, degarelix, elagolix, Valproic Acid, Anti-platelet Therapy, Tranexamic Acid, Hysterectomy, Myomectomy, Uterine Artery Embolization, Myolysis, AKT or mTOR inhibitors.

In some embodiments, the classical treatment refers to analgesics. Analgesics includes but are not limited to paracetamol, aspirin, non-steroidal anti-inflammatory drugs, codeine, morphine, tramadol, corticosteroids, antispasmodics, local anaesthetics, general anaesthetics.

As used herein, the term **"AKT"** also known as protein kinase B or PKB is well known in the art and refers to a protein serine/threonine kinase that was first discovered as an oncogene transduced by the acute transforming retrovirus (AKT-8).

As used herein, the term **"AKT inhibitor"** refers to a compound (natural or synthetic) that inhibits the signalling pathway AKT kinase (also called protein kinase B or PKB).

In a particular embodiment, the AKT inhibitors is a peptide, petptidomimetic, small organic molecule, antibody, aptamers, siRNA or antisense oligonucleotide. Several chemical classes of small-molecule AKT inhibitors with varying potencies and specificities for the different AKT isoforms have now been developed. These include phosphatidylinositol analogs, ATP-competitive small molecules, pseudosubstrate compounds, and allosteric inhibitors.

Exemplary AKT inhibitors that are contemplated by the invention include but are not limited to, for example, those as described in the following international patent applications which are hereby incorporated by reference in their entireties: aminofurazans (WO2005/019190), substituted pyrimidines (WO2008/006040), and substituted pyridines (WO2009/032653).

In a particular embodiment, the AKT inhibitor is selected from the group consisting of Perifosine KRX-0401), XL418, GSK690693, AT13148, A-443654, MK-2206 2HCl, GSK690693, Ipatasertib (GDC-0068), Capivasertib (AZD5363), SC66, PF-04691502, AT7867, Triciribine, CCT128930, A-674563, PHT-427, Miltefosine, Honokiol, TIC10 Analogue, Uprosertib (GSK2141795), TIC10, Akti-1/2, Miransertib (ARQ 092) HCl, Afuresertib (GSK2110183), AT13148, Deguelin and SC79.

In a particular embodiment, the AKT inhibitor is Miransertib.

As used herein, the term **"mTOR"** refers to mammalian target of rapamycin, kinase that in humans is encoded by the mTOR gene. mTOR is a member of the phosphatidylinositol 3-kinase-related kinase family of protein kinases (PI3K). The naturally occurring human mTOR gene has a nucleotide sequence as shown in Genbank Accession number NM_004958.3 and the naturally occurring human mTOR protein has an aminoacid sequence as shown in Genbank Accession number NP_004949.1. The murine nucleotide and amino acid sequences have also been described (Genbank Accession numbers NM_020009.2 and NP_064393.2). mTOR is involved in different pathways, including insulin, growth factors (such as IGF-1 and IGF-2), and amino acids, cellular nutrient, oxygen, and energy levels.

The term **"mTOR inhibitors"** refers to a class of drugs that inhibit mTOR. mTOR inhibitors inhibits cellular metabolism, growth, proliferation, and the formation and signaling through two protein complexes, mTORC1 and mTORC2. mTOR inhibitors are well known in the art.

In the context of the invention, mTOR inhibitor is selected from the group consisting of but not limited to: rapamycin and rapalogs (sirolimus; temsirolimus; everolimus; deforolimus); vincristine; dactolisib or BEZ235 (phase I/II of clinical trial; Novartis); or sapanisertib (phase II of clinical trial; NCI).

In a particular embodiment, the mTOR inhibitor is rapamycin.

In a particular embodiment, the mTOR inhibitor is everolimus.

As used herein the terms **"administering"** or **"administration"** refer to the act of injecting or otherwise physically delivering a substance as it exists outside the body (e.g., an inhibitor of PI3K) into the subject, such as by mucosal, intradermal, intravenous, subcutaneous, intramuscular delivery and/or any other method of physical delivery described herein or known in the art. When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof.

As used herein, the term **"administration simultaneously"** refers to administration of at least 2 or 3 active ingredients by the same route and at the same time or at substantially the same time. The term **"administration separately"** refers to an administration of at least 2 or 3 active ingredients at the same time or at substantially the same time by different routes. The term **"administration sequentially"** refers to an administration of at least 2 or 3 active ingredients at different times, the administration route being identical or different.

A **"therapeutically effective amount"** is intended for a minimal amount of active agent which is necessary to impart therapeutic benefit to a subject. For example, a "therapeutically effective amount" to a subject is such an amount which induces, ameliorates or otherwise causes an improvement in the pathological symptoms, disease progression or physiological conditions associated with or resistance to succumbing to a disorder. It will be understood that the total daily usage of the compounds of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

In a particular embodiment, the therapeutically effective amount comprises between 25mg/kg and 250 mg/kg of body weight per day.

In a particular embodiment, the therapeutically effective amount is at least 25mg/kg of body weight per day.

In a particular embodiment, the therapeutically effective amount is 250 mg/kg of body weight per day.

In a particular embodiment, the therapeutically effective amount is: 25, 50, 75, 100, 125, 150, 175, 200, 225, 250 mg/kg of body weight per day.

In a particular embodiment, the therapeutically effective amount is 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197,198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 mg/kg of body weight per day.

The PIK3CA inhibitor alone or combined with a classical treatment, as described above may be combined with pharmaceutically acceptable excipients, and optionally sustained-release matrices, such as biodegradable polymers, to form pharmaceutical compositions.

Accordingly, **in a third aspect,** the invention relates to a pharmaceutical composition comprising a PIK3CA inhibitor for use in the treatment of uterine disease as described above.

In a particular embodiment, the invention relates to a pharmaceutical composition comprising a PIK3CA inhibitor for use in the treatment of endometriosis.

In a particular embodiment, the invention relates to a pharmaceutical composition comprising a PIK3CA inhibitor for use in the treatment of myoma.

In a particular embodiment, the invention relates to a pharmaceutical composition comprising BYL719 (Alpelisib).

In a particular embodiment, the invention relates to a pharmaceutical composition a compound of formula (I), or a deuterated or tritiated form of the compound of formula (I), or a pharmaceutically acceptable salt thereof: 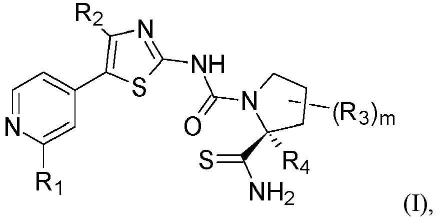 wherein:
- R₁ is a (C₁-C₆)alkyl group or a (C₃-C₆)cycloalkyl group, unsubstituted or substituted with one or more fluorine atoms;
- R₂ is chosen from:
   ▪ a hydrogen atom, and
   ▪ a (C₁-C₆)alkyl group,
- m is 0, 1 or 2;
- each R₃, when present, is independently chosen from:
   ▪ a fluorine atom,
   ▪ a (C₁-C₆)alkyl group, unsubstituted or substituted with one or more halogen atoms,
   ▪ a (C₃-C₆)cycloalkyl group unsubstituted or substituted with one or more halogen atoms,
   ▪ a hydroxy group,
   ▪ a (C₁-C₆)alkoxy group, and
   ▪ a -NRR' group, R and R' being independently chosen from a hydrogen atom and a (C₁-C₆)alkyl group,
   or two R₃, that are borne by the same carbon atom, form with the carbon atom bearing them a (C₃-C₆)cycloalkyl ring, unsubstituted or substituted with one or more fluorine atoms; and
- R₄ is chosen from:
   ▪ a fluorine atom,
   ▪ a hydrogen atom,
   ▪ a (C₁-C₆)alkyl group, unsubstituted or substituted with one or more halogen atoms, and

▪ a (C₃-C₆)cycloalkyl group, unsubstituted or substituted with one or more halogen atoms, or R₃ and R₄, when they are borne by two adjacent carbon atoms, form with the carbon atoms bearing them a (C₃-C₆)cycloalkyl ring, unsubstituted or substituted with one or more halogen atom.

In a further embodiment, the invention relates to a pharmaceutical composition comprising i) a PIK3CA inhibitor and ii) a classical treatment as described above as a combined preparation to treat uterine disease.

**"Pharmaceutically" or "pharmaceutically acceptable"** refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. The pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The polypeptide (or nucleic acid encoding thereof) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

A further object of the present invention relates to a method of screening a drug suitable for the treatment of lymphoproliferative disorder comprising i) providing a test compound and ii) determining the ability of said test compound to inhibit the activity of PI3K.

Any biological assay well known in the art could be suitable for determining the ability of the test compound to inhibit the activity of PI3K. In some embodiments, the assay first comprises determining the ability of the test compound to bind to PI3K. In some embodiments, a population of cells is then contacted and activated so as to determine the ability of the test compound to inhibit the activity of PI3K. In particular, the effect triggered by the test compound is determined relative to that of a population of immune cells incubated in parallel in the absence of the test compound or in the presence of a control agent either of which is analogous to a negative control condition. The term "control substance", "control agent", or "control compound" as used herein refers a molecule that is inert or has no activity relating to an ability to modulate a biological activity or expression. It is to be understood that test compounds capable of inhibiting the activity of PI3K, as determined using in vitro methods described herein, are likely to exhibit similar modulatory capacity in applications in vivo. Typically, the test compound is selected from the group consisting of peptides, peptidomimetics, small organic molecules, aptamers or nucleic acids. For example the test compound according to the invention may be selected from a library of compounds previously synthesised, or a library of compounds for which the structure is determined in a database, or from a library of compounds that have been synthesised de novo. In some embodiments, the test compound may be selected form small organic molecules.

The invention will be further illustrated by the following figures and examples. However, these examples and figures should not be interpreted in any way as limiting the scope of the present invention.

### FIGURES:

**Figure 1****: PIK3CA pathway is activated in adenomyosis and endometriosis lesions.** A. Representative immunofluorescence staining of P-AKTTh308 in healthy endometrium and lesions of adenomyosis. Scale bar: 20µm. B. Western blot and quantification of P-AKTSer473, P-S6RP and P-ERK in endometriosis.
**Figure 2****: A mouse model of adenomyosis.** A. Scheme of the breeding plan. B. Representative photography of PIK3CALtf female mice at the time of sacrifice demonstrating macroscopic lesions of adenomyosis. C. Ratio uterine/body weight ratio of PIK3CAWT and PIK3CALtf mice. Scale bar: 100µm. E. Representative immunofluorescence staining of Desmin and Pan-Cytokeratin in the endometrium of PIK3CAWT and PIK3CALtf mice. Scale bar: 20µm.
**Figure 3****: PIK3CA inhibition and endometriosis.** A. Table showing the mutational landscape observed in the different tissues from patients. B. Western blot of P-AKTSer473 in endometrial tissues from samples treated either with DMSO, alpelisib or ST420 at different doses. C. Quantification.
**Figure 4****: PIK3CA inhibition and myoma.** A. Western blot with quantification of P-AKTSer473 in myometrium and myoma. B. Western blot with quantification of P-AKTSer473 in myometrium and myoma from samples treated either with DMSO, alpelisib or ST420 at different doses.

### EXAMPLES:

### Method:

For this study, we interbred homozygous R26StopFLP110* (stock no. 012343) mice with Ltftm1(icre)Tdku/J mice (stock no. 026030) both obtained from the Jackson Laboratory. Following procedures previously described6, we obtained R26StopFLP110*+/- x Ltftm1 iCre+ (henceforth PIK3CALtf) and R26StopFLP110*+/- x Ltftm1 iCre-(henceforth PIK3CAWT) mice. The p110* protein expressed by R26StopFLP110* mice is a constitutively active chimera that contains the iSH2 domain of p85 fused to the NH2-terminus of p110 via a flexible glycine linker7. To generate tissue-specific p110*-transgenic mice, a cloned loxP-flanked neoR-stop cassette was inserted into a modified version of pROSA26-1 followed by the p110* coding sequence, a frt-flanked IRES-EGFP cassette and a bovine polyadenylation sequence (R26StopFLP110*)⁸.

Animals were housed at a constant ambient temperature in a 12-h light cycle and fed ad libitium with regular chow food (2018 Teklad global 18% protein rodent diets, 3.1 Kcal/g, Envigo). Animal procedures were approved by the Ministère de l'Enseignement Supérieur, de la Recherche et de l'Innovation (APAFIS N°20439-2018121913526398 and 2021110914486827). All appropriate procedures were followed to ensure animal welfare. Before the sacrifice procedure, mice were harvested over a 12-hour period.

### Magnetic resonance imaging (MRI) evaluation

All images were acquired with a 4.7-T small-animal MRI system (BioSpec USR47/40; Bruker BioSpin, Ettlingen, Germany) on the "Plateforme Imageries du Vivant, Université de Paris, PARCC, INSERM, Paris, France." Mice underwent whole body magnetic resonance imaging (MRI) using 3D T2 weighted sequences with and without fat saturation.

### Morphological analyses

Mouse tissues (uterus) were fixed in 4% paraformaldehyde and paraffin-embedded. Tissue sections (4 µm thick) were stained with hematoxylin and eosin (H&E).

### Immunohistochemistry and immunofluorescence

Paraffin-embedded mouse tissue sections were submitted to antigen retrieval protocols in either citrate or tris-EDTA buffer at high temperature (120°C) and pressure with a pressure cooker. Four-µm sections were incubated with primary antibodies (data not shown) overnight. For immunofluorescence, samples were then incubated with appropriate Alexa Fluor-conjugated secondary antibodies (Thermo Fisher Scientific) and analyzed using the Eclipse Ni-E (Nikon).

### Western blot

Tissues were crushed and then lysed in RIPA lysis buffer supplemented with phosphatase and protease inhibitors. Protein concentrations were determined through the bicinchoninic acid method (Pierce). Protein extracts were analyzed by Western blot where the transfer membrane was incubated with a primary antibody followed by the appropriate peroxidase-conjugated secondary antibody (dilution 1:10,000). Chemiluminescence were acquired using a Chemidoc MP and bands were quantitated with the Image Lab Software (Bio-Rad Laboratories).

### Tissues obtained from patients

Tissues collected from patients (endometrium, adenomyosis, endometriosis and myoma). Tissues were cultivated in media and treated during 24 hours with either DMSO, alpelisib or ST420 (Compound having formula I and/or its derivatives). Then, they were crushed and lysed in RIPA lysis buffer supplemented with phosphatase and protease inhibitors. Protein concentrations were determined through the bicinchoninic acid method (Pierce). Protein extracts were analyzed by Western blot where the transfer membrane was incubated with a primary antibody followed by the appropriate peroxidase-conjugated secondary antibody (dilution 1:10,000). Chemiluminescence were acquired using a Chemidoc MP and bands were quantitated with the Image Lab Software (Bio-Rad Laboratories).

### Patients

This study was conducted on women followed at hôpital Cochin Port Royal (Paris). In this study, written informed consent was obtained from all patients.

### Data analysis and statistics

Data were expressed as means ± SEM. Differences between experimental groups were evaluated using analysis of variance (ANOVA), followed by the Tukey-Kramer post hoc test for statistically significant differences (P < 0.05). When only two groups were compared, Mann-Whitney tests were used. The statistical analysis was performed using GraphPad Prism software (version 10.0.0).

### Results:

### PIK3CA pathway is activated in adenomyosis and endometriosis lesions

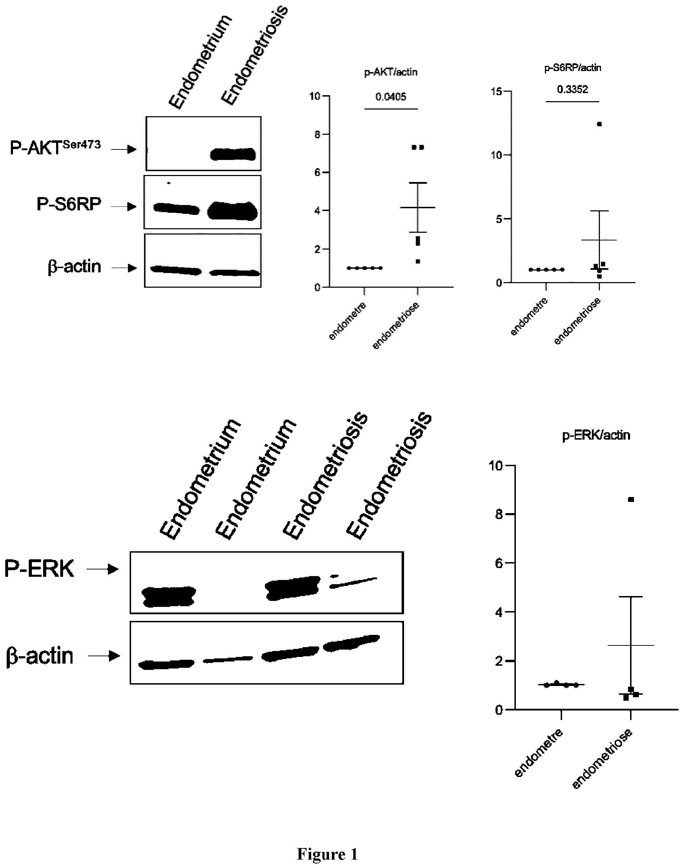

In order to understand the role played by the PIK3CA/AKT/mTOR pathways, we first explored endometriosis and adenomyosis lesions. We performed immunofluorescence experiments in normal endometrium (n= 8) and in adenomyosis and endometriosis lesions (n= 20). We observed a strong activation of AKT, a downstream effector of PIK3CA, with a high phosphorylation rate on the residue Th308 only in adenomyosis and endometriosis **(data not shown).** In order to confirm the recruitment of the AKT pathway, we conducted Western blot analysis on the protein extracted from both, heathy endometrium and lesions of endometriosis **(****Figure 1****).** Indeed, these first results suggest that PIK3CA/AKT pathway is recruited within the adenomyosis and endometriosis lesions.

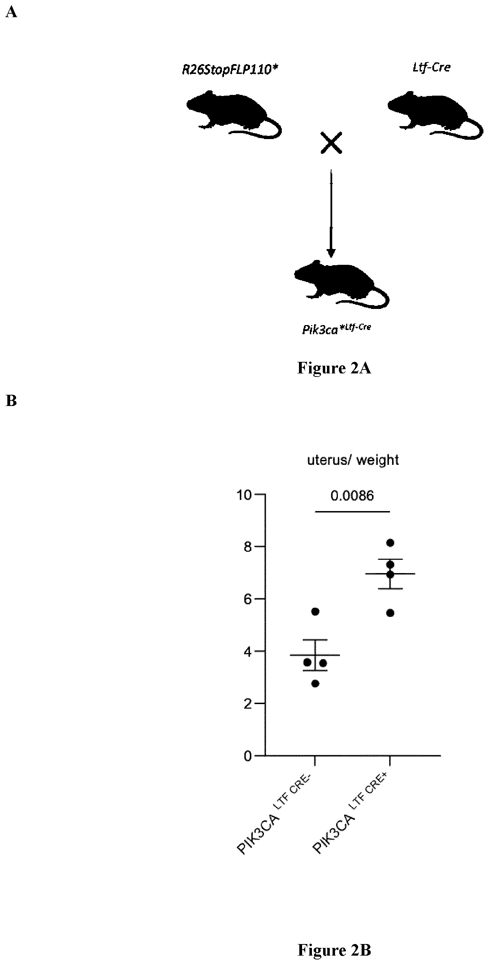

To better understand the precise role of the PI3KCA pathway in the development of the lesion of adenomyosis and endometriosis, we decided to generate a mouse model carrying a PIK3CA gain of function mutation in the endometrium. To this aim, we interbred homozygous R26StopFLP110* mice with Ltftm1(icre)Tdku/J mice. We obtained R26StopFLP110*+/- x Ltftm1 iCre+ mice (henceforth PIK3CALtf) that constitutively express an active mutant of PIK3CA, and R26StopFLP110*+/- x Ltftm1 iCre- (henceforth PIK3CAWT) mice (**Figure 2A**). Mice were born at the expected ratio. At the age of 10 weeks, mice underwent imaging exploration using magnetic resonance imaging (MRI). Pelvic MRI of the PIK3CALtf female mice revealed typical lesion of adenomyosis with T1 sequences showing high T1 signal indicating menstrual hemorrhage into the ectopic endometrial tissues and ovoid high T2 signal regions representing small areas of cystic change **(data not shown).**

Mice were then sacrificed. Compared to control mice, we observed at the macroscopic level the presence of diffuse hemorrhagic lesions in the uterus in the PIK3CALtf female mice **(data not shown).** These mice demonstrated a higher uterus weight (**Figure 2B**). At the histological level, PIK3CALtf female mice had typical lesions of adenomyosis with endometrial glands and stroma within myometrium (**Figure 2B**). We concluded that PIK3CALtf female mice developed features of adenomyosis, supporting the role of PIK3CA in the development of such lesions.

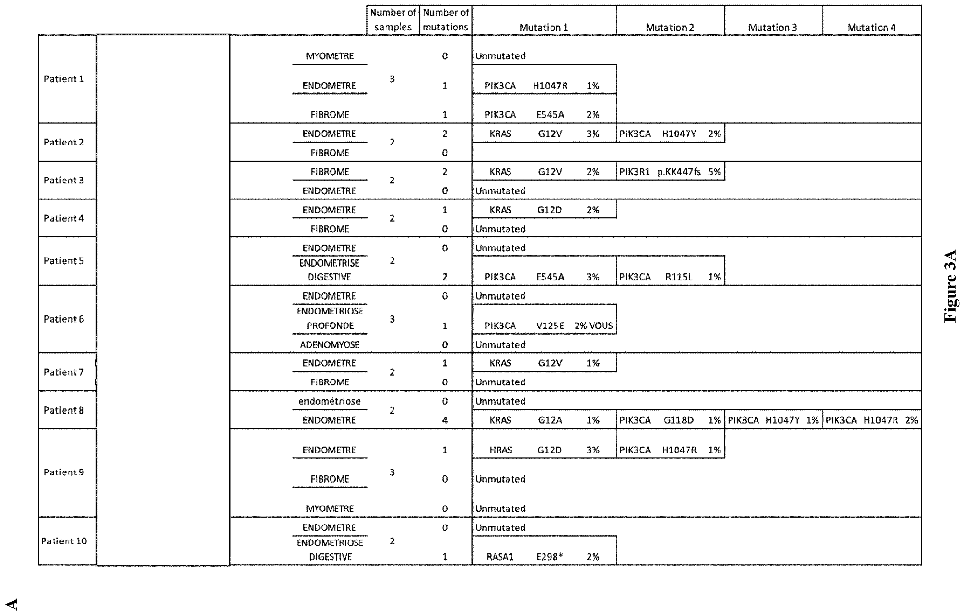

We next investigated if somatic mutation of this pathway were detectable in endometriosis and adenomyosis lesions. NGS targeted panel of 23 genes (AKT1, AKT2, AKT3, BRAF, EPHB4, GNA11, GNA14, GNAQ, HRAS, KRAS, KRIT1, MAP2K1, MAP3K3, MTOR, NRAS, PIK3CA, PIK3R1, PIK3R2, PTEN, RASA1, TEK, TSC1 and TSC2) performed in healthy endometrium (n= 7) and in lesions (n= 8) revealed the presence of somatic gain of function mutations in the healthy ones (endometrium: 85.7% with PIK3CA and KRAS) but also in few lesions (25%) consistent with recent findings that showed the presence of somatic PIK3CA and KRAS mutation in the healthy endometrium (**Figure 3A**). Indeed, we concluded that somatic mutation of these 30 genes do not explain the recruitment of the AKT pathway within the lesions.

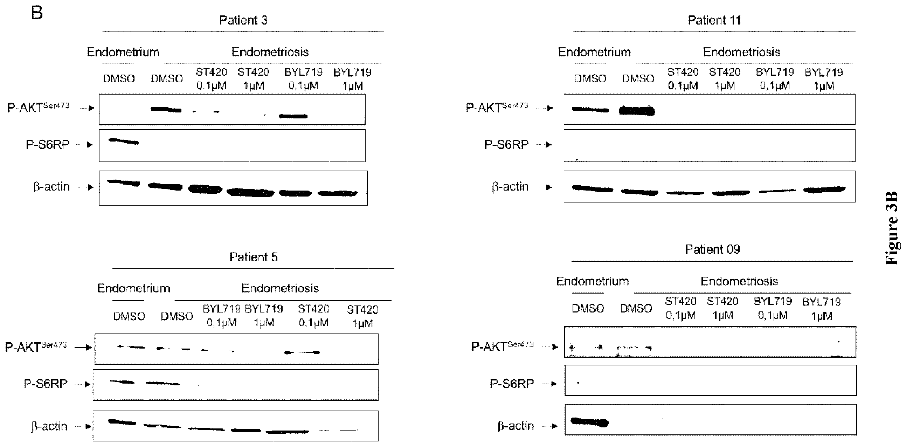

PIK3CA pharmacological inhibitors are developed for oncology and other medical situations. We wondered if these inhibitors would be able to mitigate the activation of the pathway in endometrium and adenomyosis lesions. To this aim, we collected control tissues (healthy endometrium) and different lesions of either adenomyosis or endometriosis. These samples were then exposed in vitro during 24 hours either to vehicle (DMSO) or different doses of BYI,719 or ST420 a PIK3CA inhibitor previously described. We observed that lesions treated with DMSO had a higher recruitment of the PIK3CA pathway compared to healthy endometrium (**Figure 3B**). Importantly, both inhibitors were able to shut down the phosphorylation of AKT (**Figure 3B**). We concluded that PIK3CA inhibition is a good therapeutic target for patients with endometriosis and adenomyosis.

### PIK3CA pathway is activated in myoma

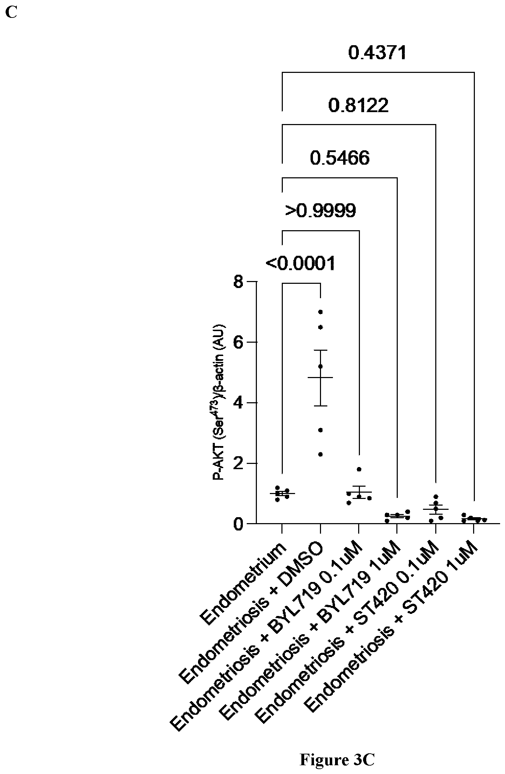

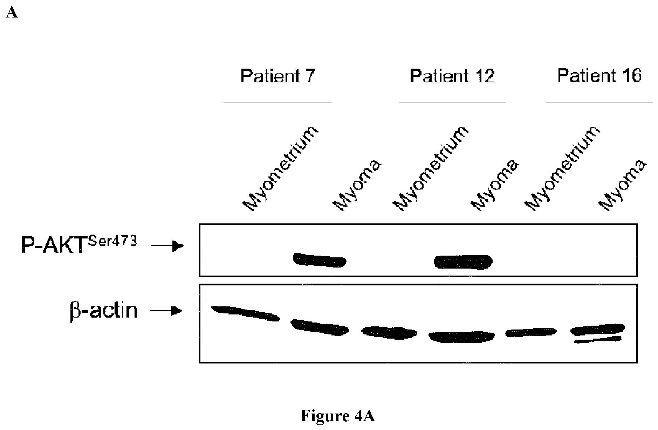

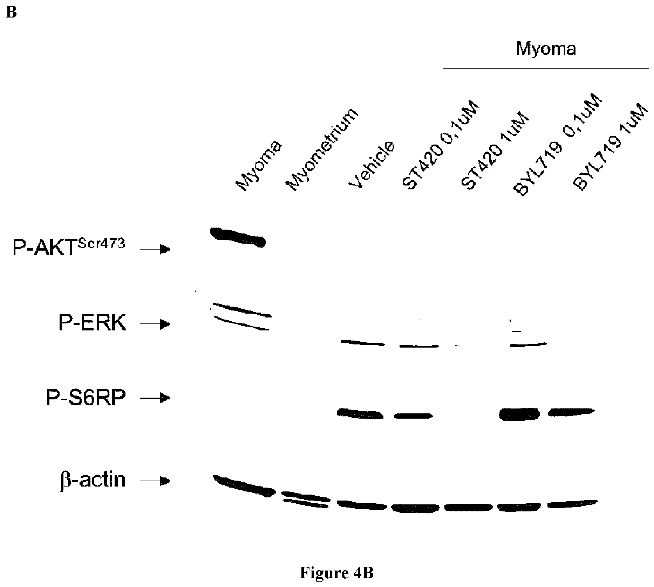

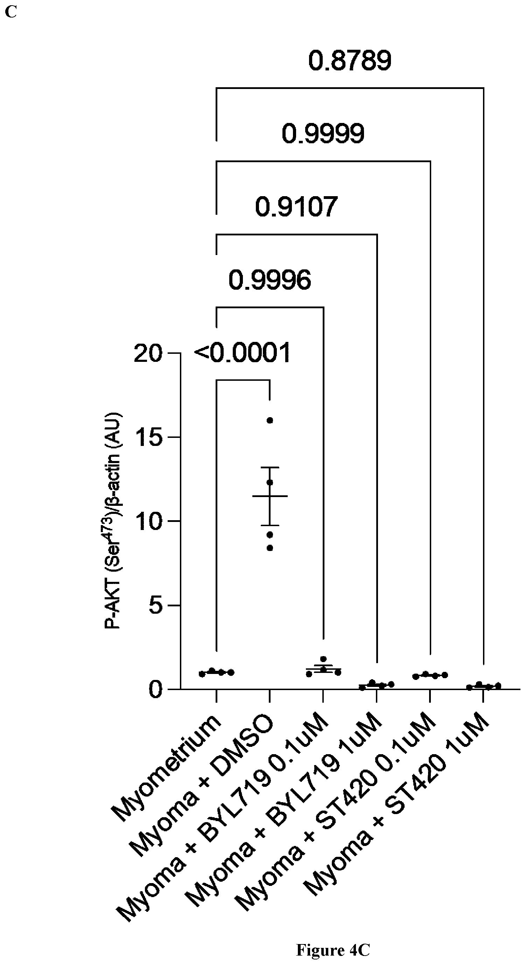

We next investigated the role of the PIK3 CA pathway in myoma (or uterine fibroids), another benign uterine condition. Myoma are small, noncancerous tumors or clusters of tumors that can grow in the walls of the uterus. Mechanisms of disease development and progression are unknown. Since they are characterized by excess of growth and proliferation, we explored the role of the PIK3CA pathway in these conditions. Western blot experiments showed that the PI3KCA pathway was recruited in myoma compared to healthy control myometrium. Targeted NGS panel (AKT1, AKT2, AKT3, BRAF, EPHB4, GNA11, GNA14, GNAQ, HRAS, KRAS, KRIT1, MAP2K1, MAP3K3, MTOR, NRAS, PIK3CA, PIK3R1, PIK3R2, PTEN, RASA1, TEK, TSC1 and TSC2) revealed the presence of somatic gain of function mutation in PIK3CA and KRAS in of 40% myoma compared to control myometrium. We finally tested the impact of PIK3CA inhibitors in vitro on different samples **(****Figure 3C****).** We observed that either alpelisib or ST420 were able to inhibit the pathway in myoma **(****Figure 4****).** We concluded that myoma demonstrate PIK3CA/AKT pathway activation with somatic mutation and are accessible to targeted treatment.

### REFERENCES:

Throughout this application, various references describe the state of the art to which this invention pertains. The disclosures of these references are hereby incorporated by reference into the present disclosure.
1. Zondervan KT, Becker CM, Missmer SA. Endometriosis. The New England journal of medicine 2020;382(13):1244-1256. DOI: 10.1056/NEJMra1810764.
2. Taylor HS, Kotlyar AM, Flores VA. Endometriosis is a chronic systemic disease: clinical challenges and novel innovations. Lancet 2021;397(10276):839-852. DOI: 10.1016/50140-673 6(21)003 89-5.
3. Andre F, Ciruelos E, Rubovszky G, et al. Alpelisib for PIK3CA-Mutated, Hormone Receptor-Positive Advanced Breast Cancer. The New England journal of medicine 2019;380(20):1929-1940. DOI: 10.1056/NEJMoa1813904.
4. Venot Q, Blanc T, Rabia SH, et al. Targeted therapy in patients with PIK3CA-related overgrowth syndrome. Nature 2018;558(7711):540-546. DOI: 10.1038/s41586-018-0217-9.
5. Canaud G, Lopez Gutierrez JC, Irvine AD, et al. Alpelisib for treatment of patients with PIK3CA-related overgrowth spectrum (PROS). Genet Med 2023;25(12):100969. (In eng). DOI: 10.1016/j.gim.2023.100969.
6. Ladraa S, Zerbib L, Bayard C, et al. PIK3CA gain-of-function mutation in adipose tissue induces metabolic reprogramming with Warburg-like effect and severe endocrine disruption. Sci Adv 2022;8(49):eade7823. DOI: 10.1126/sciadv.ade7823.
7. Klippel A, Reinhard C, Kavanaugh WM, Apell G, Escobedo MA, Williams LT. Membrane localization of phosphatidylinositol 3-kinase is sufficient to activate multiple signal-transducing kinase pathways. Molecular and cellular biology 1996;16(8):4117-27. (https://www.ncbi.nlm.nih.gov/pubmed/8754810).
8. Srinivasan L, Sasaki Y, Calado DP, et al. PI3 kinase signals BCR-dependent mature B cell survival. Cell 2009;139(3):573-86. DOI: 10.1016/j.cell.2009.08.041.

## Claims

1. A method for treating uterine disease in a subject in need thereof comprising a step of administrating the subject with a therapeutically effective amount of PIK3CA inhibitor.

2. The method according to claim 1, wherein the uterine disease is: endometriosis, myoma, adenomyosis, uterine fibroids or endometrial cancer.

3. The method according to claim 1, wherein the PIK3CA inhibitor is a peptide, peptidomimetic, small organic molecule, antibody, aptamers, siRNA or antisense oligonucleotide.

4. The method according to claim 3, wherein the PIK3CA inhibitor is: a compound of formula (I), or a deuterated or tritiated form of the compound of formula (I): 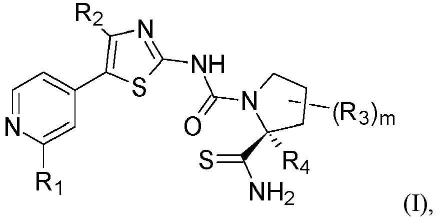 wherein:
- R₁ is a (C₁-C₆)alkyl group or a (C₃-C₆)cycloalkyl group, unsubstituted or substituted with one or more fluorine atoms;
- R₂ is chosen from:
▪ a hydrogen atom, and
▪ a (C₁-C₆)alkyl group,
- m is 0, 1 or 2;
- each R₃, when present, is independently chosen from:
▪ a fluorine atom,
▪ a (C₁-C₆)alkyl group, unsubstituted or substituted with one or more halogen atoms,
▪ a (C₃-C₆)cycloalkyl group unsubstituted or substituted with one or more halogen atoms,
▪ a hydroxy group,
▪ a (C₁-C₆)alkoxy group, and
▪ a -NRR' group, R and R' being independently chosen from a hydrogen atom and a (C₁-C₆)alkyl group,
or two R₃, that are borne by the same carbon atom, form with the carbon atom bearing them a (C₃-C₆)cycloalkyl ring, unsubstituted or substituted with one or more fluorine atoms; and
- R₄ is chosen from:
▪ a fluorine atom,
▪ a hydrogen atom,
▪ a (C₁-C₆)alkyl group, unsubstituted or substituted with one or more halogen atoms, and
▪ a (C₃-C₆)cycloalkyl group, unsubstituted or substituted with one or more halogen atoms,
or R₃ and R₄, when they are borne by two adjacent carbon atoms, form with the carbon atoms bearing them a (C₃-C₆)cycloalkyl ring, unsubstituted or substituted with one or more halogen atom, or wherein the PIK3CA inhibitor is selected from: BYL719 (Alpelisib, Novartis), GDC-0077 (Inavolisib, Genentech/Roche), TAK-117/MLN1117/INK1117 (Serabelisib) or their pharmaceutically acceptable salts.

5. The method according to claim 4, wherein the PIK3CA inhibitor is a compound of formula (I), or a deuterated or tritiated form of the compound of formula (I), or a pharmaceutically acceptable salt thereof:

6. The method according to claim 4, wherein the PIK3CA inhibitor is the compound of formula (I) or a deuterated or tritiated form of the compound of formula (I), or a pharmaceutically acceptable salt thereof, wherein R₁ is a (C₁-C₆)alkyl group, in particular a (C₁-C₄)alkyl group, and more particularly a *tert*-butyl group, unsubstituted or substituted with one or more fluorine atoms.

7. The method according to claim 4, wherein the PIK3CA inhibitor is compound of formula (I), or a deuterated or tritiated form of the compound of formula (I), or a pharmaceutically acceptable salt thereof, wherein R₂ is a (C₁-C₆)alkyl group, in particular a (C₁-C₄)alkyl group and more particularly a methyl group.

8. The method according to claim 4, wherein the PIK3CA inhibitor is the compound of formula (I), or a deuterated or tritiated form of the compound of formula (I), or a pharmaceutically acceptable salt thereof, wherein m is 1 or 2, and each R₃ is independently chosen from:
▪ a fluorine atom,
▪ a (C₁-C₆)alkyl group, in particular a (C₁-C₄)alkyl group and more particularly a methyl group, unsubstituted or substituted with one or more halogen atoms; for example a methyl group or a trifluoromethyl group;
▪ a hydroxy group, and
▪ a (C₁-C₆) alkoxy group, in particular a (C₁-C₄) alkoxy group and more particularly a methoxy group;
or, wherein m is 2, and two R₃, that are borne by the same carbon atom, form with the carbon atom bearing them a (C₃-C₆)cycloalkyl ring, in particular a cyclopropyl ring, unsubstituted or substituted with one or more fluorine atoms.

9. The method according to claim 4, wherein the PIK3CA inhibitor is the compound of formula (I) or a deuterated or tritiated form of the compound of formula (I), or a pharmaceutically acceptable salt thereof, wherein R₄ is chosen from:
▪ a hydrogen atom, and
▪ a (C₁-C₆)alkyl group, in particular a (C₁-C₄)alkyl group and more particularly a methyl group, unsubstituted or substituted with one or more halogen atoms, in particular with one or more fluorine atom.

10. The method according to claim 4, wherein the PIK3CA inhibitor is the compound of formula (I) or a deuterated or tritiated form of the compound of formula (I), or a pharmaceutically acceptable salt thereof, said compound being selected from the following compounds:
(1) (S)-2-carbamothioyl-4,4-difluoro-*N*-(4-methyl-5-(2-(1,1,1-trifluoro-2-methylpropan-2-yl)pyridin-4-yl)thiazol-2-yl)pyrrolidine-1-carboxamide,
(2) (2S,4R)-2-carbamothioyl-4-fluoro-*N*-(4-methyl-5-(2-(1,1,1-trifluoro-2-methylpropan-2-yl)pyridin-4-yl)thiazol-2-yl)pyrrolidine-1-carboxamide,
(3) (2S,4S)-2-carbamothioyl-4-fluoro-*N*-(4-methyl-5-(2-(1,1,1-trifluoro-2-methylpropan-2-yl)pyridin-4-yl)thiazol-2-yl)pyrrolidine-1-carboxamide,
(4) (S)-6-carbamothioyl-*N*-(4-methyl-5-(2-(1,1,1-trifluoro-2-methylpropan-2-yl)pyridin-4-yl)thiazol-2-yl)-1,1-difluoro-5-azaspiro[2,4]heptane-5-carboxamide,
(5) (S)-2-carbamothioyl-*N*-(4-methyl-5-(2-(1,1,1-trifluoro-2-methylpropan-2-yl)pyridin-4-yl)thiazol-2-yl)pyrrolidine-1-carboxamide,
(6) (S)-2-carbamothioyl-2-methyl-*N*-(4-methyl-5-(2-(1,1,1-trifluoro-2-methylpropan-2-yl)pyridin-4-yl)thiazol-2-yl)pyrrolidine-1-carboxamide,
(7) (2S,4R)-2-carbamothioyl-4-methoxy-*N*-(4-methyl-5-(2-(1,1,1-trifluoro-2-methylpropan-2-yl)pyridin-4-yl)thiazol-2-yl)pyrrolidine-1-carboxamide,
(8) (2S,4S)-2-carbamothioyl-4-methoxy-*N*-(4-methyl-5-(2-(1,1,1-trifluoro-2-methylpropan-2-yl)pyridin-4-yl)thiazol-2-yl)pyrrolidine-1-carboxamide,
(9) (2S,5R)-2-carbamothioyl-5-methyl-*N*-(4-methyl-5-(2-(1,1,1-trifluoro-2-methylpropan-2-yl)pyridin-4-yl)thiazol-2-yl)pyrrolidine-1-carboxamide,
(10) (2S,5S)-2-carbamothioyl-5-methyl-*N*-(4-methyl-5-(2-(1,1,1-trifluoro-2-methylpropan-2-yl)pyridin-4-yl)thiazol-2-yl)pyrrolidine-1-carboxamide,
(11) (2S,4S)-2-carbamothioyl-4-methyl-N-(4-methyl-5-(2-(1,1,1-trifluoro-2-methylpropan-2-yl)pyridin-4-yl)thiazol-2-yl)pyrrolidine-1-carboxamide,
(12) (2S,4S)-2-carbamothioyl-N-(4-methyl-5-(2-(1,1,1-trifluoro-2-methylpropan-2-yl)pyridin-4-yl)thiazol-2-yl)-4-(trifluoromethyl)pyrrolidine-1-carboxamide,
(13) (2S,4R)-2-carbamothioyl-4-hydroxy-N-(4-methyl-5-(2-(1,1,1-trifluoro-2-methylpropan-2-yl)pyridin-4-yl)thiazol-2-yl)pyrrolidine-1-carboxamide,
(14) (2S,3S)-2-carbamothioyl-3-hydroxy-N-(4-methyl-5-(2-(1,1,1-trifluoro-2-methylpropan-2-yl)pyridin-4-yl)thiazol-2-yl)pyrrolidine-1-carboxamide,
(15) (2S,3 S)-2-carbamothioyl-3-methoxy-N-(4-methyl-5-(2-(1,1,1-trifluoro-2-methylpropan-2-yl)pyridin-4-yl)thiazol-2-yl)pyrrolidine-1-carboxamide,
(16) (S)-6-carbamothioyl-N-(4-methyl-5-(2-(1,1,1-trifluoro-2-methylpropan-2-yl)pyridin-4-yl)thiazol-2-yl)-5-azaspiro[2.4]heptane-5-carboxamide,
(17) (2S,4S)-2-carbamothioyl-4-hydroxy-N-(4-methyl-5-(2-(1,1,1-trifluoro-2-methylpropan-2-yl)pyridin-4-yl)thiazol-2-yl)pyrrolidine-1-carboxamide;
(18) (S)-2-carbamothioyl-N-(4-methyl-5-(2-tert-butyl-pyridin-4-yl)thiazol-2-yl)pyrrolidine-1-carboxamide,
(19) (2S,4S)-2-carbamothioyl-4-fluoro-N (4-methyl-5-(2-tert-butyl-pyridin-4-yl)thiazol-2-yl)pyrrolidine-1-carboxamide, and
(20) (S)-6-carbamothioyl-N-(4-methyl-5-(2-tert-butyl-pyridin-4-yl)thiazol-2-yl)-5-azaspiro[2.4]heptane-5-carboxamide.

11. The method according to claim 1, wherein the PIK3CA inhibitor is the compound of formula (I-A) or (II-A), or any of their pharmaceutically acceptable salts 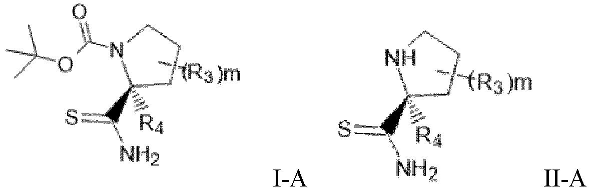 wherein R₃, R₄ and m are as defined in any one of claims 1, 4 and 5; or R₃ represents a protected hydroxy group, for example a hydroxy group protected with a *tert-*butyldiphenylsilyl ether (tBDPS) protecting group.

12. The method according to claims 4 and claim 11, wherein the PIK3CA inhibitor is selected from the following compounds:
(21) tert-butyl (S)-2-carbamothioyl-4,4-difluoropyrrolidine-1-carboxylate;
(23) tert-butyl (2S,4S)-2-carbamothioyl-4-fluoropyrrolidine-1-carboxylate,
(24) tert-butyl (6S)-6-carbamothioyl-1,1-difluoro-5-azaspiro[2.4]heptane-5-carboxylate, (26) tert-butyl (S)-2-carbamothioyl-2-methylpyrrolidine-1-carboxylate,
(28) tert-butyl (2S,4S)-2-carbamothioyl-4-methoxypyrrolidine-1-carboxylate,
(29) tert-butyl (2S,SR)-2-carbamothioyl-5-methylpyrrolidine-1-carboxylate,
(30) tert-butyl (2S,5S)-2-carbamothioyl-5-methylpyrrolidine-1-carboxylate,
(31) tert-butyl (2S,4S)-2-carbamothioyl-4-methylpyrrolidine-1-carboxylate,
(32) tert-butyl (2S,4S)-2-carbamothioyl-4-(trifluoromethyl)pyrrolidine-1-carboxylate,
(34) tert-butyl (2S,3S)-3-((tert-butyldiphenylsilyl)oxy)-2-carbamothioylpyrrolidine-1-carboxylate,
(35) tert-butyl (2S,3S)-2-carbamothioyl-3-methoxypyrrolidine-1-carboxylate,
(36) tert-butyl (S)-6-carbamothioyl-5-azaspiro[2.4]heptane-5-carboxylate, and
(37) tert-butyl (2S,4S)-4-((tert-butyldiphenylsilyl)oxy)-2-carbamothioylpyrrolidine-1-carboxylate;
or a deuterated or tritiated form of the compound of formula (I), or pharmaceutically acceptable salts thereof.

13. A PIK3CA inhibitor and a classical treatment as a combined preparation for simultaneous, separate or sequential use in the treatment of uterine disease in a subject in need thereof.

14. The PIK3CA inhibitor and a classical treatment as a combined preparation for use according to claim 13 wherein, the classical treatment is: gonadotropin releasing hormone analogues (GnRH analogues), Progestins (norethindrone acetate (NETA)), Levonorgestrel-releasing intrauterine system (LNG-IUS); combined oral contraceptives, nonsteroidal anti-inflammatory drugs, Aromatase Inhibitors (letrozole [Femara], anastrozole [Arimidex], fadrozole, Selective Progesterone Receptor Modulators, chlormadinone acetate, cyproterone acetate, danazol, desogestrel, drospirenone, dienogest, 5α-dihydroprogesterone, ethanediol acetate, ethynodiol diacetate, etonogestrel, gestodene, estretol, 17-hydroxyprogesterone, levomefolate, levonorgestrel, medroxyprogesterone acetate (17α-hydroxy-6α-methylprogesterone acetate), megestrol, megestrol acetate (17αacetoxy-6-dehydro-6-methylprogesterone), nestorone, nomegestrol acetate, norethindrone, norethindrone acetate, norethynodrel, norgestimate, norgestrel, progesterone, tanaproget, trimegestone, danazol, leuprolide, leuprolide acetate, goserelin, goserelin acetate, histrelin, histrelin acetate, triptorelin, nafarelin, degarelix, elagolix,Valproic Acid, Anti-platelet Therapy, Tranexamic Acid, Hysterectomy, Myomectomy, Uterine Artery Embolization, Myolysis, AKT or mTOR inhibitors.

15. A pharmaceutical composition comprising a PIK3CA inhibitor for use in the treatment of uterine disease.

16. The pharmaceutical composition according to claim 15 wherein the inhibitor of PIK3CA is BYL719 (Alpelisib).

17. The pharmaceutical composition according to claim 15 wherein the inhibitor of PIK3CA is compound of formula (I), or a deuterated or tritiated form of the compound of formula (I), or a pharmaceutically acceptable salt thereof: 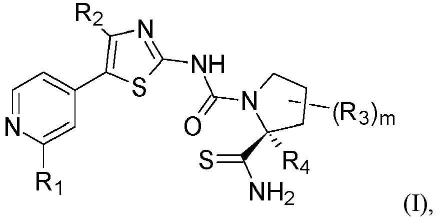 wherein:
- R₁ is a (C₁-C₆)alkyl group or a (C₃-C₆)cycloalkyl group, unsubstituted or substituted with one or more fluorine atoms;
- R₂ is chosen from:
▪ a hydrogen atom, and
▪ a (C₁-C₆)alkyl group,
- m is 0, 1 or 2;
- each R₃, when present, is independently chosen from:
▪ a fluorine atom,
▪ a (C₁-C₆)alkyl group, unsubstituted or substituted with one or more halogen atoms,
▪ a (C₃-C₆)cycloalkyl group unsubstituted or substituted with one or more halogen atoms,
▪ a hydroxy group,
▪ a (C₁-C₆)alkoxy group, and
▪ a -NRR' group, R and R' being independently chosen from a hydrogen atom and a
(C₁-C₆)alkyl group,
or two R₃, that are borne by the same carbon atom, form with the carbon atom bearing them a (C₃-C₆)cycloalkyl ring, unsubstituted or substituted with one or more fluorine atoms; and
- R₄ is chosen from:
▪ a fluorine atom,
▪ a hydrogen atom,
▪ a (C₁-C₆)alkyl group, unsubstituted or substituted with one or more halogen atoms, and
▪ a (C₃-C₆)cycloalkyl group, unsubstituted or substituted with one or more halogen atoms,or R₃ and R₄, when they are borne by two adjacent carbon atoms, form with the carbon atoms bearing them a (C₃-C₆)cycloalkyl ring, unsubstituted or substituted with one or more halogen atom.
